# EUROPEAN PATENT APPLICATION

(11) **EP 4 483 828 A2**
(43) Date of publication of application: **01.01.2025**
(21) Application number: 24213973.1
(22) Date of filing: 30.09.2020
(51) Int. Cl.: A61B 18/14

(54) **ELECTRODE APPARATUS FOR DIAGNOSIS OF ARRHYTHMIAS**

(30) Priority: 29.05.2020 US 202063031955 P; 16.07.2020 US 202063052553 P; 23.09.2020 US 202017029752; 23.09.2020 US 202017029890
(62) Divisional of application: 20199175.9
(71) Applicant: Biosense Webster (Israel) Ltd., 2066717 Yokneam (IL)
(72) Inventor: SALAZAR, Henry F., Irvine, 92618 (US); TOBEY, Dustin R., Irvine, 92618 (US); BASU, Shubhayu, Irvine, 92618 (US); THALER, Sean D., Irvine, 92618 (US); WONG, Joseph R., Irvine, 92618 (US); GHIDOLI, Daniele, Irvine, 92618 (US); VAN NIEKERK, Pieter Emmelius, Irvine, 92618 (US); MALINARIC, Jamie Lynn, Irvine, 92618 (US); FUENTES-ORTEGA, Cesar, Irvine, 92618 (US)
(74) Representative: Carpmaels & Ransford LLP

(57) **Abstract**

An apparatus includes an end effector having loop members with electrodes thereon and is usable with catheter-based systems to measure or provide electrical signals. The end effector can include three loop members that are non-coplanar when expanded unconstrained that become contiguous to a planar surface when the loop members are deflected against the surface, a mechanical linkage that joins the loop members at a distal vertex of the end effector, electrodes having surface treatment to enhance surface roughness of the electrodes, twisted pair electrode wires, a bonded spine cover, and/or any combination thereof.

## Description

### CROSS REFERENCE TO RELATED APPLICATIONS

This application claims the benefits of priority under the Paris Convention as well as 35 USC §§ 119 and 120 to prior filed US Provisional Patent Application SN 63/031,955 (Attorney Docket No. 253757.000039), titled as "ELECTRODE APPARATUS FOR DIAGNOSIS OF ARRHYTHMIAS" and filed on May 29, 2020 and US Provisional Patent Application SN 63/052,553 (Attorney Docket No. 253757.000040) titled as "INTRALUMINAL REFERENCE ELECTRODE FOR CARDIOVASCULAR TREATMENT APPARATUS" filed on July 16, 2020, which priority applications are hereby incorporated by reference as set forth in full herein.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to provide high-density signal maps through the use of several electrodes sensing electrical activity of tissue in an area on the order of a square centimeter. For mapping within an atria or a ventricle (for example, an apex of a ventricle), it is desirable for a catheter to collect larger amounts of data signals within shorter time spans. It is also desirable for such a catheter to be adaptable to different tissue surfaces, for example, flat, curved, irregular or nonplanar surface tissue and be collapsible for atraumatic advancement and withdrawal through a patient's vasculature.

### SUMMARY

Example apparatuses disclosed herein are generally usable with catheter-based systems to measure or provide electrical signals within the heart and surrounding vasculature. Example apparatuses generally include an end effector having loop members with electrodes thereon. The end effector can include features which provide improved and/or alternative diagnostic or treatment options compared to existing end effectors. Such features can include three loop members that are non-coplanar when expanded unconstrained that become contiguous to a planar surface when the spines are deflected against the surface, a mechanical linkage that joins three loop members of the end effector, electrodes having surface treatment to enhance surface roughness of the electrodes, twisted pair electrode wires, a bonded spine cover, and/or any combination thereof.

An example apparatus includes an elongated shaft and an end effector. The elongated shaft has a proximal portion and a distal portion and is configured to be manipulated at the proximal portion to position the distal portion into the heart of a patient. The elongated shaft defines a longitudinal axis of the apparatus. The end effector is disposed near the distal portion of the elongated shaft. The end effector includes three loop members overlapping at a common distal vertex along the longitudinal axis. Each of the three loop members includes a respective pair of ends affixed to the distal portion of the elongated shaft.

The end effector can be configured to expand to an unconstrained configuration when unconstrained to define three different planes for the respective three loop members. When the distal portion of the elongated shaft is deflected at an angle relative to the longitudinal axis, a majority of a length of each of the loop members becomes contiguous to a planar surface, thereby moving the end effector into in a flattened configuration. The three loop members are thereby movable to a flattened configuration when the loop members are positioned against a planar surface.

The majority of the length each of the loop members can be non-coplanar with the majority of the length of at least one of the other loop members when the end effector is in the unconstrained configuration.

Each of the loop members respectively can include a support frame extending through a respective loop member. Each support frame can be affixed to the distal portion of the elongated shaft where each end of the respective pair of ends of the respective loop member is affixed to the distal portion of the elongates shaft. Each of the respective support frames can define a respective looped path of its respective loop member when the end effector is in the unconstrained configuration. Each of the respective support frames can include a respective cross sectional shape orthogonal to the respective looped path, each of the respective cross sectional shapes varying along the respective looped path.

Each of the respective support frames can include a serrated edge engaged to the distal portion of the elongated shaft.

Each of the respective support frames can include a respective pair of parallel segments. When the end effector is in the flattened configuration, a majority of the length of each segment of the pairs of parallel segments can be coplanar with each other. When the end effector is in the unconstrained configuration, the majority of the length of at least one segment of the pairs of parallel segments can be non-coplanar with the majority of the respective length of at least one of the other segments of the pairs of parallel segments.

Each of the support frames can include a respective connecting segment extending between the respective pair of parallel segments and overlapping at the distal vertex with the respective connecting segment of each of the other respective support frames.

The apparatus can further include a mechanical linkage binding the three loop members at the common distal vertex.

The mechanical linkage can include a rectangular or ovular shape having an opening through which the three loop members extend and a side comprising a seam. Alternatively, the mechanical linkage can include four contiguous sides, i.e. lacking a seam. Alternatively, the mechanical linkage can include three openings through which a respective loop member extends. At least one of the three openings can have a substantially circular shape while at least one other of the three openings has an oblong shape.

As an alternative to having a rectangular or ovular shape, the mechanical linkage can have a cylindrical shape with three passageways therethrough. The three passageways can each have a respective loop member of the three loop members extending therethrough. Some or all of the three loop members can include a respective tubular housing surrounding the respective support frame that also extends through the respective passageway. Some or all of the looped members can lack an outer housing where they extend through the respective passageway (e.g. support members can be bare at the distal vertex. Some or all of the support members which are bare where they pass through the respective passageway can have a respective tubular housing surrounding the respective support frame elsewhere along the looped path.

As an alternative to having a rectangular, ovular, or cylindrical shape, the mechanical linkage can be shaped in the form of a tapered ring having an annular opening through which the three loop members extend and a tapered height extending across a diameter of annular opening.

The apparatus can further include multiple electrodes affixed to the three loop members. Each electrode can have a surface characterized by roughness parameter *Ra* representing an arithmetical mean deviation of a profile of the surface, where *Ra* measures from about 0.3 micrometers to about 0.4 micrometers.

The apparatus can further include wires connected to electrodes carried by the loop members. The wires can be bundled together within the loop members. The apparatus can further include wire pairs twisted about each other within the end effector. Each wire pair can be electrically connected to a respective pair of electrodes.

As a first possible loop member housing configuration, the three loop members can each respectively include an inner tube housing, an outer tubular housing, and electrical conductors. The inner tubular housing can surround at least a portion of the respective support frame. The outer tubular housing can surround at least a portion of the inner tubular housing and can be bonded to the inner tubular housing. The electrical conductors can be disposed at least partially within the outer tubular housing and outside of the inner tubular housing. Each of the three loop members can further respectively include an irrigation tube positioned within the outer tubular housing and outside of the inner tubular housing.

As a second possible loop member housing configuration, alternative to the first possible configuration, the three loop members can each respectively include a tubular housing, electrical conductors, and an irrigation tube. Each respective tubular housing can surround the respective support frame and include at least two lumens therethrough such that the respective support frame extends through a first lumen of the two lumens. The electrical conductors can be disposed in a second lumen of the at least two lumens separate from the first lumen. The irrigation tube can also be disposed in the second lumen.

As a third possible loop member housing configuration, alternative to the first and second possible configurations, the three loop members can each respectively include a tubular housing, electrical conductors, and an irrigation lumen. Each tubular housing can surround the respective support frame and include at least three lumens therethrough. The respective support frame can extend through a first lumen of the at least three lumens. The electrical conductors can be disposed in a second lumen of the at least three lumens separate from the first lumen. The third lumen of the at least three lumens separate from the first and second lumens can be configured for irrigation. The third lumen can be configured to irrigate direction and/or can include an irrigation tube.

The first, second, and third loop member configurations are combinable such that one loop member in the apparatus can have one of the first, second, and third possible loop member housing configurations and another loop member of the apparatus can have a different configuration.

The apparatus can further include at least one pull wire extending through the elongated shaft and attached to the distal portion of the elongated shaft so that when the pull wire is retracted toward the proximal portion relative to the elongated shaft, the distal portion and the end effector are bent at an angle with respect to the longitudinal axis.

An example method can include one or more of the following steps presented in no particular order. A first loop member, a second loop member, and a third loop member can each respectively be shaped to each form a respective loop. A respective pair of ends of each of the first loop member, the second loop member, and the third loop member can be coupled to a distal portion of an elongated shaft. The first loop member, the second loop member, and the third loop member can be overlapped at a common distal vertex distal to the distal portion of the elongated shaft such that when the first, second, and third loop members are unconstrained, a majority of the first loop member is non-coplanar with a majority of at least one of the second loop member and the third loop member. The majority of the first loop member, the majority of the second loop member, and the majority of the third loop member can be pressed in contact with a planar surface via manipulation of the elongated shaft. The first, second and third loop members can be placed into contact with a planar surface to align the majority of the first loop member, the majority of the second loop member, and the majority of the third loop member with the planar surface.

The method can further include positioning the first loop member, second loop member, third loop member, and distal portion of the elongated shaft within an intravascular catheter (or guiding sheath) while a proximal portion of the elongated shaft extends proximally from the intravascular catheter.

The method can further include moving the first loop member, second loop member, and third loop member out of a distal end of the catheter via manipulation of the proximal portion of the elongated shaft.

The method can further include pressing the majority of the first loop member, the majority of the second loop member, and the majority of the third loop member in contact with the planar surface via manipulation of the proximal portion of the elongated shaft.

The method can further include shaping a first support frame to define a first looped path such that the first support frame comprises a cross sectional shape orthogonal to the first looped path that varies along the first looped path. The method can further include positioning the first support frame in the first loop member. The method can further include shaping a second support frame to define a second looped path such that the second support frame comprises a cross sectional shape orthogonal to the second looped path that varies along the second looped path. The method can further include positioning the second support frame in the second loop member. The method can further include shaping a third support frame to define a third looped path such that the third support frame comprises a cross sectional shape orthogonal to the third looped path that varies along the third looped path. The method can further include positioning the third support frame in the third loop member.

The method can further include affixing the first, second, and third support frames to the distal portion of the elongated shaft at each end of the respective pair of ends of the first loop member, the second loop member, and the third loop member.

The method can further include engaging a serrated edge of each of the first, second, and third support frames to the distal portion of the elongated shaft.

The method can further include positioning the first support frame, the second support frame, and the third support frame such that the first looped path defines a first plane intersecting at least one of a second plane defined by the second looped path and a third plane defined by the third looped path.

The method can further include shaping a first pair of parallel segments in the first support frame, a second pair of parallel segments in the second support frame, and a third pair of parallel segments in the third support frame. The method can further include moving a majority of a length of each segment of the first pair of parallel segments, the second pair of parallel segments, and the third pair of parallel segments into alignment parallel to the planar surface via manipulation of the elongated shaft.

The method can further include shaping a first connecting segment extending between the first pair of parallel segments, a second connecting segment extending between the second pair of parallel segments, and a third connecting segment extending between the third pair of parallel segments.

The method can further include mechanically binding the three loop members at the distal vertex.

The method can further include forming a clip having two ends and a partially wrapped shape having an opening sized to receive the first, second, and third loop members. The method can further include moving the first, second, and third loop members through the opening into the partially wrapped shape. The method can further include moving the two ends of the clip to collapse the opening. The method can further include confining the first, second, the third loop members at the distal vertex with the clip.

As an alternative to confining the first, second, and third loop members with the clip, the method can include confining the first, second, the third loop members at the distal vertex within an opening of an alternative mechanical linkage having a contiguous perimeter.

As another alternative, the method can include forming another alternative mechanical linkage having a rectangular or ovular shape having a first opening, a second opening, and a third opening. The method can further include positioning the first loop member in the first opening, the second loop member in the second opening, and the third loop member in the third opening. The method can further include linking the first, second, and third loop members at the distal vertex with the mechanical linkage. The method can further include forming the first opening to have a substantially circular shape. The method can further include forming at least one of the second opening and the third opening to have an oblong shape.

As another alternative, the method can include forming another alternative mechanical linkage having a cylindrical shape with a first passageway therethrough, a second passageway therethrough, and a third passageway therethrough. The method can further include positioning the first loop member in the first passageway, the second loop member in the second passageway, and the third loop member in the third passageway. The method can further include linking the first, second, and third loop members at the distal vertex with the mechanical linkage. The method can further include surrounding each of the first loop member, the second loop member, and the third loop member with a respective tubular housing. The method can further include positioning each of the respective tubular housings respectively through the first passageway, the second passageway, and the third passageway. As an alternative to positioning the respective tubular housings through the first, second, and third passageways, the method can include surrounding each of the first loop member, the second loop member, and the third loop member with a respective tubular housing excepting at least a respective portion of the first loop member, the second loop member, and the third loop member where the respective loop member extends through the respective passageway.

As another alternative to confining the first, second, and third loop members with the clip, the method can include confining the first, second, the third loop members at the distal vertex within an opening of a mechanical linkage including a tapered ring having an annular opening through which the first, second, the third loop members extend and a height tapering across a diameter of annular opening.

The method can further include affixing a plurality of electrodes to the three loop members. The method can further include abrading at least a portion of a surface of each electrode of the plurality of electrodes. The method can further include swaging each electrode of the plurality of electrodes.

The method can further include electrically connecting wires to electrodes carried by the first, second, and third loop members. The method can further include bundling the wires within the first, second, and third loop members.

The method can further include at least partially surrounding the first support frame in an inner tubular housing. The method can further include positioning a plurality of electrical conductors adjacent to the first support frame and outside of the inner tubular housing. The method can further include at least partially surrounding the inner tubular housing and the plurality of electrical conductors with an outer tubular housing. The method can further include bonding the outer tubular housing to the inner tubular housing.

As an alternative to the steps including the inner tubular housing and the outer tubular housing, the method can include positioning the first support frame within a first lumen of a tubular housing having at least two lumens therethrough. The method can further include positioning a plurality of electrical conductors within a second lumen of the tubular housing, the second lumen being separate from the first lumen. The method can further include positioning an irrigation tube in the second lumen.

As another alternative to the steps including the inner tubular housing and the outer tubular housing, the method can further include positioning the first support frame within a first lumen of a tubular housing having at least three lumens therethrough. The method can further include positioning a plurality of electrical conductors within a second lumen of the tubular housing, the second lumen being separate from the first lumen. The method can further include positioning an irrigation tube within a third lumen of the tubular housing, the third lumen being separate from the first lumen and the second lumen.

Another example method can include the following steps presented in no particular order. A distal portion of an elongated shaft and an end effector extending distally from the distal portion can be moved through a catheter (or guiding sheath) to the heart. The end effector can be moved from a distal end of the catheter via manipulation of a proximal portion of the elongated shaft. The end effector can be expanded to an unconstrained configuration distal to the distal end of the catheter such that the end effector has three loop members overlapping in three layers at a common distal vertex in the unconstrained configuration. The end effector can be pressed into cardiac tissue via manipulation of the proximal portion of the elongated shaft. A majority of each of the loop members can be conformed to the cardiac tissue as a result of pressing the end effector into cardiac tissue.

The method can further include expanding the end effector in the unconstrained configuration such that the majority of each of the three loop members are non-coplanar with at least one of the other three loop members.

The method can further include bending three support frames, each extending through a respective loop member of the three loop members and affixed to the distal portion of the elongated shaft, as a result of pressing the end effector into cardiac tissue.

The method can further include positioning the three support frames such that each respective support frame has a respective pair of parallel segments. The method can further include aligning a majority of each length of each segment of each of the respective pair of parallel segments parallel to the cardiac tissue as a result of pressing the end effector into cardiac tissue. The method can further include bending the three support frames on either side of the majority of each length of each segment of each of the respective pair of parallel segments as a result of pressing the end effector into cardiac tissue.

The method can further include bending the three support frames along thinner segments of the support frame, the thinner segments having a cross sectional area measuring less than a cross sectional area of the majority of each length of each segment of each of the respective pair of parallel segments.

The method can further include positioning the three support frames such that each respective support frame includes a respective connecting segment extending between the respective pair of parallel segments and overlapping, at the distal vertex, the respective connecting segment of each of the other respective support frames.

The method can further include maintaining the overlapping of the three loop members at the distal vertex with a mechanical linkage positioned at the distal vertex.

The method can further include receiving electrical signals having signals representing noise of less than 0.03 mV from electrodes positioned on the end effector and in contact with the cardiac tissue.

### BRIEF DESCRIPTION OF THE DRAWINGS

Figure 1A is an illustration of a catheter having an end effector at a distal portion of the catheter and a proximal handle at a proximal portion of the catheter according to aspects of the present invention.
Figures 1B and 1C and illustrations of orthogonal side plan views of a variation of the apparatus illustrated in Figure 1A according to aspects of the present invention.
Figures 2A through 2C are illustrations of an intermediate section and distal portion of a shaft of the catheter in greater detail according to aspects of the present invention.
Figures 3A and 3B are illustrations of a front and side view of the end effector according to aspects of the present invention.
Figures 4A through 4D are illustrations depicting orientation of loop members of the end effector according to aspects of the present invention.
Figure 5A is an illustration depicting electrode spacing and dimensions on the end effector according to aspects of the present invention.
Figure 5B is another illustration of the end effector including a mechanical linkage according to aspects of the present invention.
Figures 6A and 6B are illustrations of views of the end effector pressed to a planar surface according to aspects of the present invention.
Figure 7 is an illustration of an intermediate section of the shaft of the catheter deflected with a puller wire.
Figures 8A and 8B are illustrations of views of support frames of the end effector in an unconstrained configuration according to aspects of the present invention.
Figures 8C and 8D are illustrations of the support frames of Figures 8A and 8B being pressed to a surface according to aspects of the present invention.
Figures 9A and 9B are illustrations of example support frames that vary in cross section along a respective looped path of each support frame according to aspects of the present invention.
Figure 10A is an illustration of example support frames of the end effector according to aspects of the present invention.
Figures 10B through 10E are illustrations of cross sectional areas of the example support frame as indicated in Figure 10A.
Figures 10F and 10G are illustrations of possible transition schemes between regions of a support frame of the end effector according to aspects of the present invention.
Figure 11A is an illustration of an asymmetrical support frame of the end effector according to aspects of the present invention.
Figures 11B and 11C are illustrations of cross sections of the asymmetrical support frame as indicated in Figure 11A.
Figure 12A is an illustration of a symmetrical support frame of the end effector according to aspects of the present invention.
Figures 12B and 12C are illustrations of cross sections of the symmetrical support frame as indicated in Figure 12A.
Figure 12D is an illustration of a detailed section of the symmetrical support frame as indicated in Figure 12A.
Figure 13A is an illustration of another asymmetrical support frame of the end effector according to aspects of the present invention.
Figures 13B and 13C are illustrations of cross sections of the asymmetrical support frame as indicated in Figure 13A.
Figures 13D and 13E are illustrations of a detailed sections of the asymmetrical support frame as indicated in Figure 13A.
Figure 14A is an illustration of a symmetrical support frame of the end effector according to aspects of the present invention.
Figures 14B and 14C are illustrations of cross sections of the symmetrical support frame as indicated in Figure 14A.
Figures 14D and 14E are illustrations of a detailed sections of the symmetrical support frame as indicated in Figure 14A.
Figure 15 is an illustration of crimped or serrated features at end of the support frames according to aspects of the present invention.
Figures 16A through 16D are illustrations of deformation of the support frames as a result of application of various forces according to aspects of the present invention.
Figures 17A through 17D are illustrations of an example rectangular or ovular mechanical linkage having a closable single opening and used for joining the loop members according to aspects of the present invention.
Figures 18A through 18C are illustrations of an example rectangular or ovular mechanical linkage having a single opening and used for joining the loop members according to aspects of the present invention.
Figures 19A through 19C are illustrations of an example rectangular or ovular mechanical linkage having three openings and used for joining the loop members according to aspects of the present invention.
Figures 20A through 20C are illustrations of an example cylindrical mechanical linkage having three passageways and used for joining support members of the loop members according to aspects of the present invention.
Figures 21A through 21C are illustrations of an example cylindrical mechanical linkage having three passageways and used for joining the loop members with tubular housings over the support members according to aspects of the present invention.
Figures 22A through 22C are illustrations of example mechanical linkages according to aspects of the present invention.
Figures 23A and 23B are illustrations of an example loop member having an inner and outer housing surrounding the support frame according to aspects of the present invention.
Figures 24A through 24C are illustrations of an alternative example loop member having a tubular housing having two or three lumens therethrough in which the support frame, conductive wires, and an irrigation tube are positioned according to aspects of the present invention.
Figures 25A through 25F are illustration of example end effector electrodes with roughened surfaces according to aspects of the present invention.

### DETAILED DESCRIPTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the pertinent art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

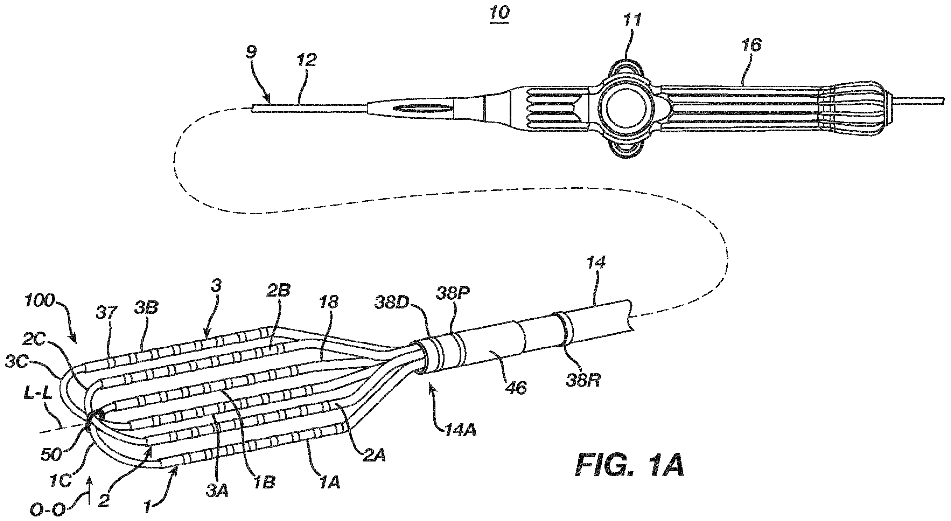

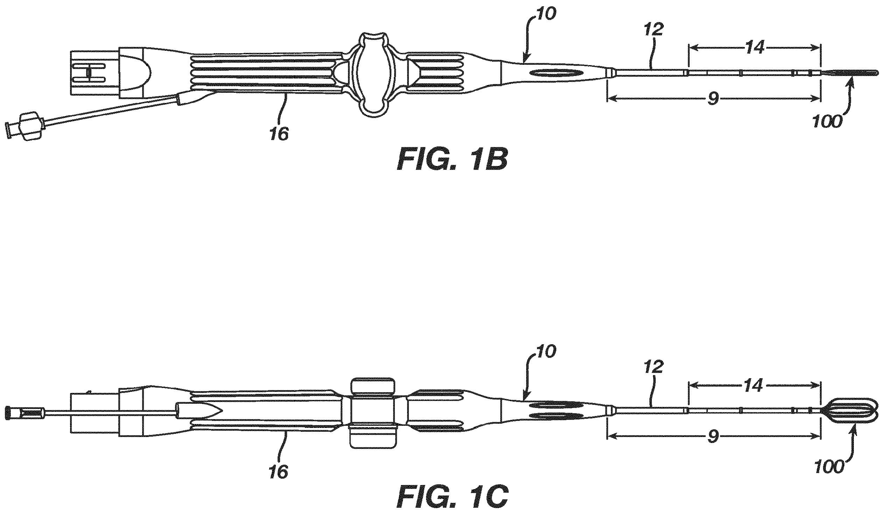

Figure 1A illustrates an example apparatus 10 having an elongated shaft 9, a distal electrode assembly or end effector 100, and a deflection control handle 16. The shaft 9 is preferably a tubular member. Figures 1B and 1C and illustrations of orthogonal side plan views of a variation of the apparatus 10 illustrated in Figure 1A. The apparatus 10 can have several design variations to while including novel aspects illustrated herein. The apparatus 10 is presented for illustration purposes only and is not intended to be limiting.

The elongated shaft 9 has a proximal portion 12 in the shape of an elongated catheter body, an intermediate deflection section 14, and distal portion 14A. The deflection control handle 16 is attached to the proximal end of the catheter body 12. The distal portion 14A of the shaft is coupled to the end effector 100 via a connector tubing 46. The elongated shaft 9 forms a tubular catheter body sized and otherwise configured to traverse vasculature. The end effector 100 has a plurality of loop members 1, 2, 3 that overlap at a common distal vertex and are joined at the distal vertex with a mechanical linkage 50.

When the device is unconstrained and aligned, the proximal portion 12, intermediate section 14, distal portion 14A, and end effector 100 are generally aligned along a longitudinal axis L-L. The intermediate section 14 can be configured to bend to deflect the distal portion 14A and end effector 100 from the longitudinal axis L-L.

The end effector 100 can be collapsed (compressed toward the longitudinal axis L-L) to fit within a guiding sheath or catheter (not illustrated). The shaft 9 can be pushed distally to move the end effector 100 distally through the guiding sheath. The end effector 100 can be moved to exit a distal end of the guiding sheath via manipulation of the shaft 9 and/or control handle 16. An example of a suitable guiding sheath for this purpose is the Preface Braided Guiding Sheath, commercially available from Biosense Webster, Inc. (Irvine, California, USA).

The end effector 100 has first, second and third loop members 1, 2, and 3. Each loop member 1, 2, 3 has two spines 1A, 1B, 2A, 2B, 3A, 3B and a connector 1C, 2C, 3C that connects the two spines of the respective loop member 1, 2, 3. Spines 1A, 1B of a first loop member 1 are connected by a first connector 1C; spines 2A, 2B of a second loop member 2 are connected by a second connector 2C; and spines 3A, 3B of a third loop member 3 are connected by a third connector 3C. The connectors 1C, 2C, 3C are preferably arcuate members as illustrated.

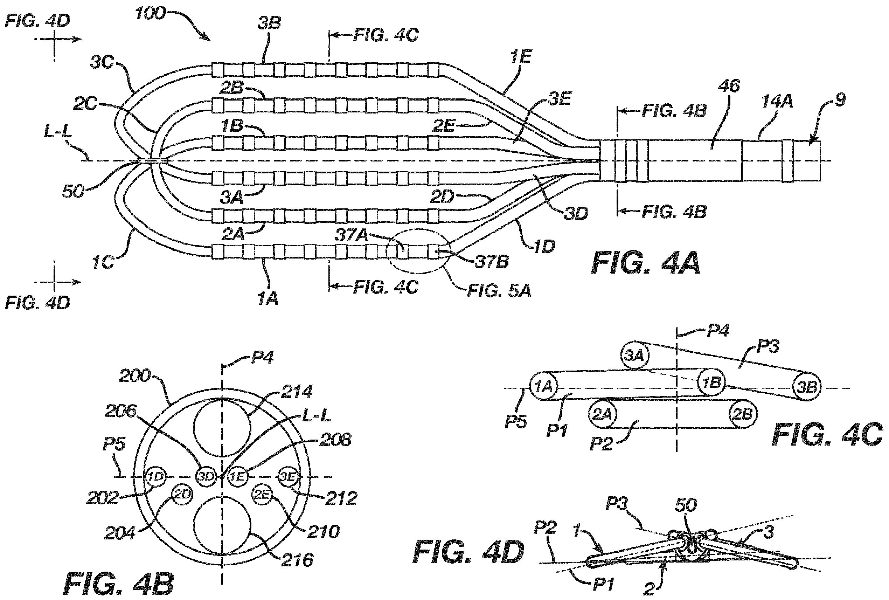

For each loop member 1, 2, 3 the spines 1A, 1B, 2A, 2B, 3A, 3B in the respective pair of spines can be substantially parallel to each other along a majority of their respective lengths when the end effector 100 is expanded in an unconstrained configuration as illustrated in Figure 1A. Preferably, all spines in the end effector are parallel to each other along the majority of their respective lengths when the end effector 100 is in the unconstrained configuration. Even when all spines are parallel, the spines are not necessarily all coplanar as described in greater detail elsewhere herein, for instance in relation to Figures 4A through 4C.

Each spine 1A, 1B, 2A, 2B, 3A or 3B can have a length ranging between about 5 and 50 mm, preferably about 10 and 35 mm, and more preferably about 28 mm. The parallel portions of each spine 1A, 1B, 2A, 2B, 3A, 3B can be spaced apart from each other by a distance ranging between about 1mm and 20 mm, preferably about 2 and 10 mm, and more preferably about 4 mm. Each spine 1A, 1A, 1B, 2A, 2B, 3A, 3B preferably carries at least eight electrodes per spine member. The end effector preferably includes six spines as illustrated. With eight electrodes on six spines, the end effector 100 includes forty-eight electrodes.

A distal electrode 38D and a proximal electrode 38P are positioned near the distal portion 14A of the shaft 9. The electrodes 38D and 38P can be configured to cooperate (e.g. by masking of a portion of one electrode and masking a different portion on the other electrode) to define a referential electrode (an electrode that is not in contact with tissues). One or more impedance sensing electrodes 38R can be configured to allow for location sensing via impedance location sensing technique, as described in US Patent Nos. 5,944,022; 5,983,126; and 6,445,864, of which a copy is provided in the priority US Provisional Patent Application 63/031,955 and incorporated herein by reference.

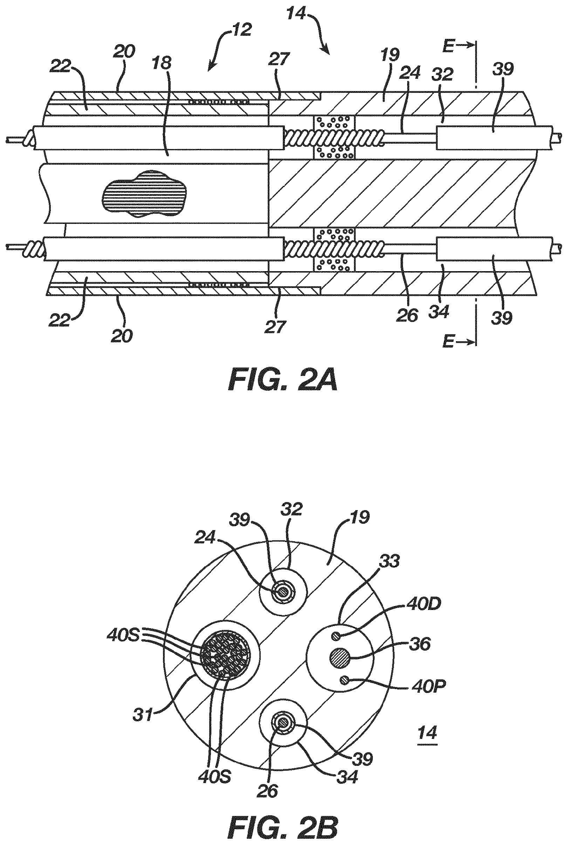

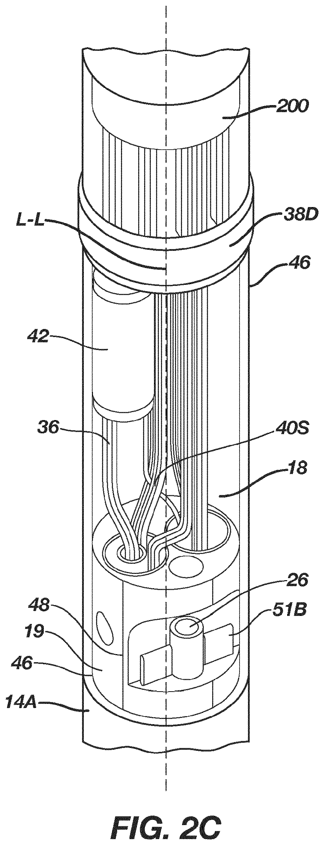

Figures 2A through 2C illustrates the intermediate section 14 and distal portion 14A of the shaft 9 of the apparatus in greater detail. Figure 2A is a cross sectional view, along the longitudinal axis L-L, of the elongated shaft 9 at the interface between the proximal portion 12 and intermediate section 14. Figure 2B is a cross sectional view of the intermediate section 14 orthogonal to the longitudinal axis L-L. Figure 2C is an isometric view of the distal portion 14A and connector tubing 46 with certain components illustrated as transparent.

As illustrated in Figure 2A, the catheter body 12 can be an elongated tubular construction having a single axial passage or central lumen 18. The catheter body 12 is flexible, i.e., bendable, but substantially non-compressible along its length. The catheter body 12 can be of any suitable construction and made of any suitable material. In some embodiments, the catheter body 12 has an outer wall 20 made of polyurethane or PEBAX. The outer wall 20 may include an imbedded braided mesh of stainless steel or the like to increase torsional stiffness of the catheter body 12 so that, when the control handle 16 is rotated, the intermediate section 14 will rotate in a corresponding manner.

The outer diameter of the catheter body 12 is preferably no more than about 8 French, more preferably about 7 French. The thickness of the outer wall 20 is thin enough so that the central lumen 18 can accommodate at least one puller wire, one or more lead wires, and any other desired wires, cables or tubes. If desired, the inner surface of the outer wall 20 is lined with a stiffening tube 22 to provide improved torsional stability. In some embodiments, the outer wall 20 has an outer diameter of from about 0.090 inch to about 0.094 inch (from about 2.3 mm to about 2.4 mm) and an inner diameter of from about 0.061 inch to about 0.065 inch (from about 1.5 mm to about 1.7 mm).

As illustrated particularly in Figure 2B, the intermediate section 14 can include a shorter section of tubing 19 having multiple lumens, for example, four off-axis lumens 31, 32, 33 and 34. The first lumen 31 carries a plurality of lead wires 40S for ring electrodes 37 carried on the spines 1A, 1B, 2A, 2B, 3A, 3B. The second lumen 32 carries a first puller wire 24. The third lumen 33 carries a cable 36 for an electromagnetic position sensor 42 and lead wires 40D and 40P for distal and proximal ring electrodes 38D and 38P carried on the catheter proximally of the end effector 100. Electromagnetic location sensing technique is described in US Patent Nos. 5,391,199; 5,443,489; 5,558,091; 6,172,499; 6,590,963; and 6,788,967 of which a copy is provided in the priority US Provisional Patent Application 63/031,955 and incorporated herein by reference. The magnetic location sensor 42 can be utilized with impedance sensing electrode 38R in a hybrid magnetic and impedance position sensing technique known as ACL described in US Patent Nos. 7,536,218; 7,756,567; 7,848,787; 7,869,865; and 8,456,182, of which a copy is provided in the priority US Provisional Patent Application 63/031,955 and incorporated herein by reference.

The fourth lumen 34 (for example, diametrically opposite of the second lumen 32 as illustrated) carries a second puller wire 26. The tubing 19 is made of a suitable non-toxic material that is preferably more flexible than the catheter body 12. One suitable material for the tubing 19 is braided polyurethane, i.e., polyurethane with an embedded mesh of braided stainless steel or the like. The size of each lumen is sufficient to house the lead wires, puller wires, the cable and any other components.

The useful length of the catheter shaft 9, i.e., that portion of the apparatus 10 that can be inserted into the body excluding the end effector, can vary as desired. Preferably the useful length ranges from about 110 cm to about 120 cm. The length of the intermediate section 14 is a relatively smaller portion of the useful length, and preferably ranges from about 3.5 cm to about 10 cm, more preferably from about 5 cm to about 6.5 cm.

Catheter body proximal portion 12 can be attached to the intermediate section 14 as shown and described in Figures 2A and 2B of US Patent No. 9,820,664, of which a copy is provided in the priority US Provisional Patent Application 63/031,955 and incorporated herein by reference. If desired, a spacer (not shown) can be located within the catheter body 12 between the distal end of the stiffening tube (if provided) and the proximal end of the intermediate section 14. The spacer can provide a transition in flexibility at the junction of the catheter body 12 and intermediate section 14, which can allow this junction to bend smoothly without folding or kinking. A catheter having such a spacer is described in US Pat. No. 5,964,757 of which a copy is provided in the priority US Provisional Patent Application 63/031,955 and incorporated herein by reference.

The distal portion 14A of the shaft 9 can be substantially contiguous with the intermediate section 14 such that the intermediate section comprises the distal portion 14A; the distal portion being distinguished from the intermediate section 14 by the positioning of one or more (optional) ring electrodes 38R. As referred to herein, the distal portion 14A of the shaft 9 can therefore correspond to a distal portion of the intermediate section 14.

As illustrated in Figure 2C, the distal portion 14A of the shaft 9 is coupled to the end effector 100 with a connector tubing 46. The connector tubing 46 includes an insert for connection of loop members 1, 2, 3 to provide electrical connection through the intermediate portion 14 of the catheter body. The connector tubing 46 can be affixed to the distal portion 14A of the catheter by glue or the like.

The connector tubing 46 can be shaped to house various components such as an electromagnetic position sensor, a puller wire anchor, ring electrodes 38D, 38P, etc. The connector tubing 46 can include a central lumen 48 to house various components. An outer circumferential notch 27 (Figure 2A) in the distal end of the tubing 19 that receives the inner surface of the proximal end of the connector tubing 46 can be used to attach the connector tubing 46 and the intermediate section 14 (distal portion 14A of shaft 9). The intermediate section 14 and connector tubing 46 are attached by glue or the like.

The connector tubing 46 can house various components, including an electromagnetic position sensor 42, and a distal anchor bar for a first puller wire 24 and another anchor bar 51B for a second puller wire 26. Only the anchor 51B for second puller wire 26 is visible in Figure 2C. The anchor bar for the first puller wire can be configured as a mirror image to the illustrated puller wire anchor bar 51B. Carried on the outer surface of the tubing 19 near the distal end of the intermediate deflection section 14 (distal portion 14A of the shaft 9), a distal ring electrode 38D is connected to lead wire formed in the side wall of the tubing 19. The distal end of the lead wire is welded or otherwise attached to the distal ring electrode 38D as known in the art.

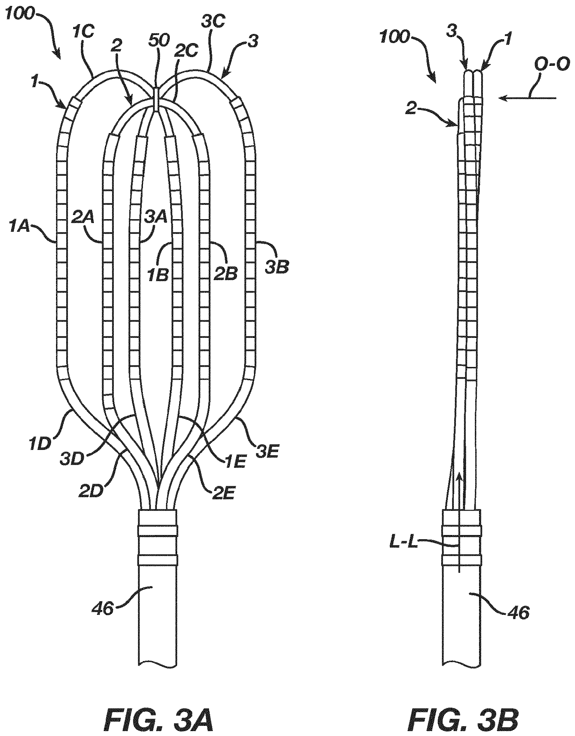

Figures 3A and 3B are illustrations of a front and side view of the end effector 100. The three loop members 1, 2, 3 overlap at a common distal vertex 50 along the longitudinal axis L-L. Each of the loop members 1, 2, 3, respectively include proximal end segments 1D, 2D, 3D, 1E, 2E, 3E affixed to the distal portion 14A of the elongated shaft 9 of the apparatus 10.

The end effector 100 is in an unconstrained configuration as illustrated in Figures 3A and 3B. As better visualized in the side view of Figure 3B, when the end effector is unconstrained, the loop members 1, 2, 3 are not coplanar with each other. Figure 3B also illustrates an orthogonal axis O-O orthogonal to the longitudinal axis L-L and approximately orthogonal to the front view of the end effector 100.

Figures 4A through 4D are illustrations depicting orientation of loop members of the end effector. Figures 4B and 4C are cross sectional views of the end effector 100 and as indicated in Figure 4A. Figure 4D is a view of the end effector 100 looking proximally from a distal end of the end effector 100 as indicated in Figure 4A.

Figure 4B illustrates a cross sectional view through the connector 46. The connector 46 includes a tubular insert 200 that has its center coinciding with the longitudinal axis L-L. Orthogonal planes P4 and P5 are in alignment with the longitudinal axis to define four quadrants in the insert 200. A parallel reference plane P5 is approximately parallel to the front view of the end effector 100 illustrated in Figure 3A. An orthogonal reference plane P4 is approximately orthogonal to the parallel reference plane P5 and approximately parallel to the orthogonal axis O-O. Apertures 202, 204, 206, 208, 210, 212 of the insert 200 are sized, positioned, and otherwise configured for insertion of respective end segments 1D, 2D, 3D, 1E, 2E, 3E. Openings 214, 216 are disposed on orthogonal plane P4 for insertion of puller wires or electrical wires as well as any other components to and from the end effector 100. Components which traverse the apertures 202, 204, 206, 208, 210, 212 and openings 214, 216 are not illustrated in Figure 4B for the purposes of illustration.

With this arrangement of apertures 202, 204, 206, 208, 210 and 212, loop members 1, 2 and 3 are arrayed in a non-coplanar unconstrained arrangement, shown in the sectional view of Figure 4C (as viewed from the proximal end), whereby loop 3 defines a plane P3 (demarcated by spines 3A and 3B with connector 3C) that intersects orthogonal plane P4 with loop 1 having a plane P1 (demarcated by spines 1A and 1B with connector 1C) that intersects both orthogonal planes P4 and P5 and loop 2 having a plane P2 (demarcated by spines 2A and 2B and connector 2C) that intersects orthogonal plane P4 and is substantially parallel to orthogonal plane P5. Figure 4D is a view of the distal end of the end effector looking proximally. In particular, loop 1 (defined by spines 1A, 1B and connector 1C) is arrayed to define plane P1 that is contiguous to or extend through spines 1A, 1B and loop 1C whereas spines 2A, 2B and connector 2C of loop 2 are arrayed to define a plane P2 that intersects with plane P1. Spines 3A, 3B and connector 3C of loop 3 are arrayed to define a plane P3 that intersects with both planes P1 and P2. The planes P1, P2, and P3 defined by the respective loops 1, 2 and 3 are configured so that the loops P1, P2 and P3 are not contiguous to or arrayed such that a common plane passes through the loops. Thus, planes P1, P2 and P3 are non-parallel and intersects each other. It is noted that the longitudinal axis L-L may be contiguous to the second plane P2. In alternative embodiment, longitudinal axis L-L may be disposed in between a region bounded by planes P1, P2 and P3.

Figures 4A through 4D are one example of non-coplanar arrangement of loop members 1, 2, 3 in an end effector. There are numerous possible arrangements non-coplanar arrangements of loop members which can result in an end effector having the general appearance of the illustrated end effector 100.

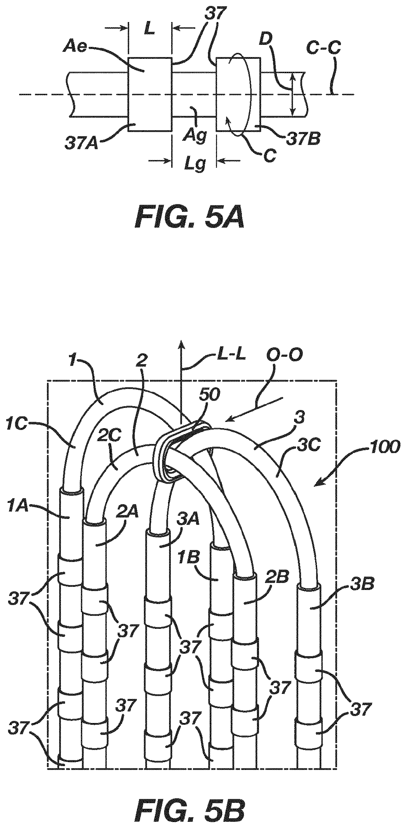

Figure 5A is an illustration depicting electrode spacing and dimensions on the end effector. The electrodes 37 can include one or more pairs of closely-spaced bipolar microelectrodes 37A, 37B which are configured to pick up electrocardiogram signals from tissue. In the present example, the microelectrodes 37A, 37B of a pair has a separation space gap distance (Lg) therebetween of approximately 1 mm to 200 microns and preferably no greater than about 200 microns. Each electrode 37A, 37B has an electrode area (Ae) and electrode length (L). The electrode length can be from about 2 mm to about 0.5 mm. Each spine electrode 37 preferably has a length of 1 mm to 0.5 mm. The electrodes 37 as illustrated are cylindrical such that the electrode area is calculated as a produce of the circumference (C) and length (L) of the electrode. The spine has a diameter (D).

Additionally, or alternatively, the microelectrodes 37A, 37B need not completely circumscribe the respective loop 1, 2, 3; in which case the microelectrodes 37A, 37B can have a rectangular shape that is rectilinear or arced having a width (W) such that the electrode area (Ae) is a produce of the electrode length (L) and width (W), the width being the arc length when the rectangular shape is arced. In examples where the electrode pair configurations are in shapes other than rectilinear, rectangular, or cylindrical, a conversion factor CF may be used to determine the appropriate gap distance between the electrodes based on the known area of either one of the pair of electrodes. The conversion factor CF may range from about 2 to 0.1 in the inverse of the same root dimensional unit as the planar area of an electrode. In one example, where the planar area of one electrode is about 0.08 squared-mm, the smallest gap distance (Lg) along the longitudinal axis extending through both electrodes can be determined by applying the conversion factor CF (in the inverse of the same root dimensional unit of the area or mm) to arrive at a gap distance Lg of about 100 microns. In another example where the area of one electrode is 0.24 squared-mm, the conversion factor CF (in the inverse of the same root dimensional unit or mm⁻¹) can be 1.25mm⁻¹ or less, giving the range of the smallest gap distance Lg from about 300 microns to about 24 microns. Regardless of the shape of the electrodes, a preferred conversion factor CF is about 0.8 (in the inverse of the same root dimensional unit for the electrode area).

Figure 5B is another illustration of the end effector 100 including a mechanical linkage 50. At least one pair of closely-spaced bipolar microelectrodes 37A, 37B is provided on each spine 1A, 2A, 3A, 1B, 2B, 3B in the present example. More particularly, each spine 1A, 2A, 3A, 1B, 2B, 3B carries four pairs of bipolar microelectrodes 37 corresponding to eight microelectrodes 37 per spine. This number may be varied as desired. Figure 5B also illustrates a clip 50 coupling connectors 1C, 2C, 3C together in a single connection point. The clip 50 functions to maintain a spatially fixed arrangement between the loops 1, 2, 3 at the common distal vertex.

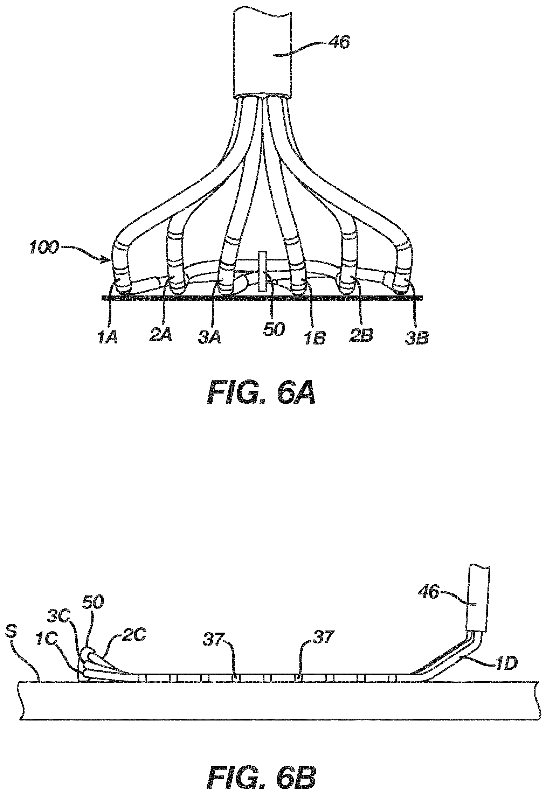

Figures 6A and 6B are illustrations of views of the end effector pressed to a planar surface S. In the illustrated example, the loop members 1, 2, 3 can be pressed to the planar surface S via manipulation of the shaft 9 of the device. More specifically, when the end effector 100 is positioned within a patient, manipulation of the catheter body 12 and the control handle 16 can be used to position the end effector 100 against a surface within a wall of an internal cavity of the patient such internal walls of the heart and/or blood vessels. When the end effector 100 is positioned against the planar surface S, a majority of each length of each spine 1A, 2A, 3A, 1B, 2B, 3B can become contiguous and aligned to the planar surface. Further, when the end effector 100 is positioned against the planar surface S, a majority of each length of each spine 1A, 2A, 3A, 1B, 2B, 3B can become aligned with a majority of each length of the other spines. The surface S need not necessarily be planar in order for the spines 1A, 2A, 3A, 1B, 2B, 3B to become contiguous and aligned to the surface. The end effector 100 may be able to conform to a curved surface, for instance.

As illustrated in Figure 6B, when the majority of each spine 1A, 2A, 3A, 1B, 2B, 3B is pressed to the surface S, the connecting segments 1C, 2C, 3C can be stacked on top of the surface S at the distal vertex at the linkage 50. A first connecting segment 1C nearest to the surface S can be separated from the surface S by the linkage 50. A second connecting segment 3C stacked onto the first connecting segment 1C can be separated from the surface S by the linkage 50 and the first connecting segment 1C. A third connecting segment 2C can be separated from the surface S by the linkage 50 and the first and second connecting segments 1C, 3C. Therefore, at least a portion of each connecting segment 1C, 2C, 3C can be separated from the planar surface S when the majority of each spine 1A, 2A, 3A, 1B, 2B, 3B is pressed to the planar surface. Alternatively, the linkage 50 can be inset into the first connecting segment 1C such that the first connecting segment is substantially contiguous to the planar surface S. In that case, only the second and third connecting segments 3C, 2C are separated from the planar surface S at the distal vertex.

Proximal segments 1D, 2D, 3D, 1E, 2E, 3E of the loop members 1, 2, 3 can be bent such that at least a portion of each of the proximal segments curves away from the surface S.

When the majority of each spine 1A, 2A, 3A, 1B, 2B, 3B is pressed to the surface S, at least some of the electrodes 37 on each spine can be in contact with the surface S. In some examples, every electrode 37 on each spine can be in contact with the surface 37.

When the majority of each spine 1A, 2A, 3A, 1B, 2B, 3B is pressed to the surface S, the majority of each respective length of each loop member can become contiguous to the surface S, where the respective length of each loop member includes the length of the respective loop member's spines 1A, 2A, 3A, 1B, 2B, 3B, connectors 1C, 2C, 3C, and proximal segments 1D, 2D, 3D, 1E, 2E, 3E (distal to the connector tubing 46).

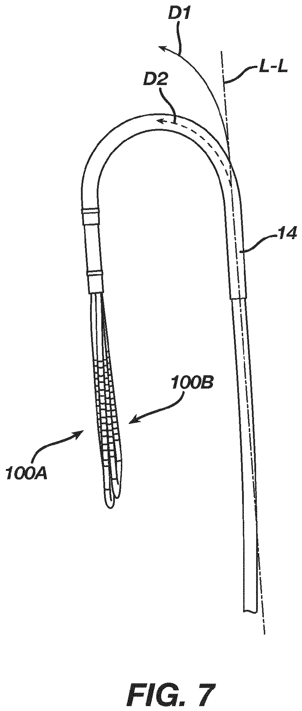

Figure 7 is an illustration of the intermediate section 14 of the shaft of the catheter deflected at approximately 360°. The end effector 100 has a first side 100A and a second side 100B. This allows the user to place first side 100A (or 100B) against the tissue surface, with at least the intermediate section 14 (if not also a distal portion of the catheter body 12) generally perpendicular to the tissue surface, and actuates the control handle to deflect the intermediate deflection section 14 to arrive at various deflections or radii of curvature (e.g., arrows D1 and D2) such that the second side 100B deflects back toward the catheter body 12. Being positioned as such may allow dragging of the second side 100B of the end effector 100 including the loop members 1, 2, 3 across the tissue surface as the intermediate section 14 is deflecting. The intermediate section can be deflected via manipulation of the puller wires 24, 26 illustrated in Figures 2A through 2C. The puller wires 24, 26 can be two separate tensile members or parts of a single tensile member. In some examples, the puller wires 24, 26 can be actuated for bi-directional deflection of the intermediate section 14. The puller wires 24, 26 can be actuated by mechanisms in the control handle 16 that are responsive to a thumb control knob or a deflection control knob 11. Suitable control handles are disclosed in US Patent Nos. 6,123,699; 6,171,277; 6,183,435; 6,183,463; 6,198,974; 6,210,407 and 6,267,746, of which a copy is provided in the priority US Provisional Patent Application 63/031,955 and incorporated herein by reference.

Details of the construction of puller wires including anchor via T-bars 51B (see Figure 2C) at the intermediate section 14, as known in the art and described in, for example, US Patent Nos. 8,603,069 and 9,820,664, of which a copy is provided in the priority US Provisional Patent Application 63/031,955 and incorporated herein by reference. The puller wires 24 and 26 can be made of any suitable metal, such as stainless steel or Nitinol. The puller wires 24, 26 are preferably coated with TEFLON or the like. The coating imparts lubricity to the puller wires. The puller wires preferably have a diameter ranging from about 0.006 to about 0.010 inch.

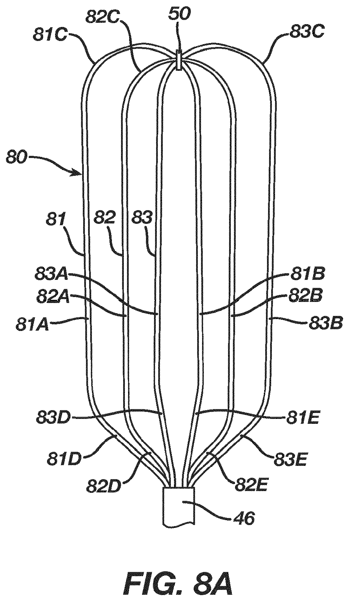

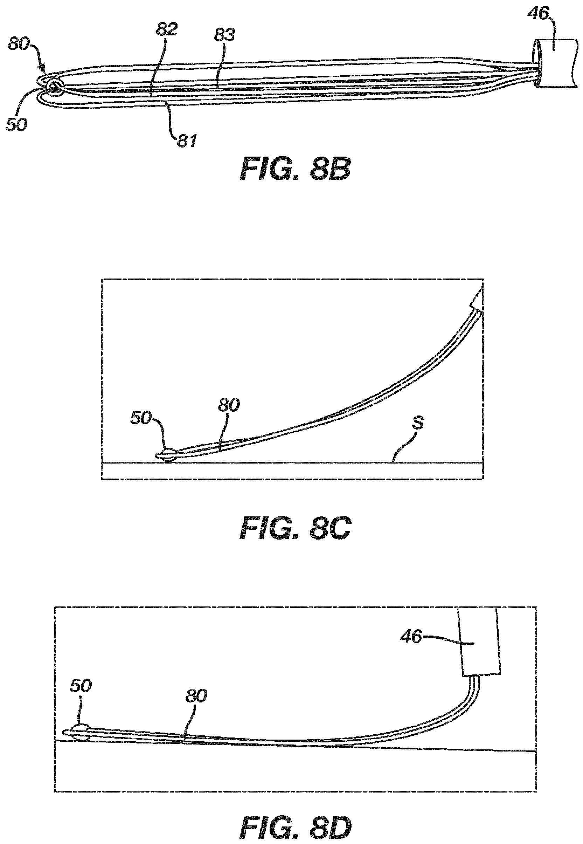

Figures 8A through 8D are illustrations of a support frame assembly 80 of the end effector 100. Figures 8A and 8B illustrate the support frame assembly 80 in an unconstrained configuration. Figures 8C and 8D illustrate the support frame assembly 80 being pressed to a surface S. When the end effector 100 is assembled, the loop members 1, 2, 3 each includes a respective support frame 81, 82, 83. The support frame assembly 80 extends into the connector tubing 46 to mechanically affix the loop members 1, 2, 3 to the shaft 9. The support frames 81, 82, 83 provide structural integrity for the loop members 1, 2, 3. The support frames 81, 82, 83 can include plastic or metal cut-off sheets, plastic or metal round wire, plastic or metal square wire, or other suitable biocompatible material. In the preferred embodiments, the support frame are made from shape memory material such as, for example, nitinol. For the purposes of testing and illustration, the support frame assembly 80 is joined at a distal vertex with mechanical linkage 50. In the assembled end effector 100, the support frames 81, 82, 83 can be assembled by virtue of a mechanical linkage affixed to an outer housing of the loop members 1, 2, 3 or a direct linkage between the support frames 81, 82, 83.

When the end effector is unconstrained, each of the respective support frames 81, 82, 83 defines a respective looped path of its respective loop member 1, 2, 3 as illustrated in Figure 8A. Each support frame 81, 82, 83 includes respective parallel segments 81A, 82A, 83A, 81B, 82B, 83B that extend through corresponding spines 1A, 2A, 3A, 1B, 2B, 3B of the end effector 100. Each support frame 81, 82, 83 includes respective proximal segments 81D, 82D, 83D, 81E, 82E, 83E that extend through corresponding proximal segments 1D, 2D, 3D, 1E, 2E, 3E of respective loop members 1, 2, 3. The proximal segments 81D, 82D, 83D, 81E, 82E, 83E extend into the connector tubing 46 to join the end effector 100 to the shaft 9. Each support frame 81, 82, 83 includes a respective connecting segments 81C, 82C, 83C that extends between the respective pair of parallel segments 81A, 82A, 83A, 81B, 82B, 83B and through the respective connecting segment 1C, 2C, 3C of the respective loop member 1, 2, 3.

When the end effector is unconstrained, at least one of the parallel segments 81A, 82A, 83A, 81B, 82B, 83B is not aligned in a common plane with other parallel segments. Said another way, at least one of the looped paths is non-coplanar with one or both of the other looped paths. The pair of parallel segments 81A, 82A, 83A, 81B, 82B, 83B for each support frame 81, 82, 83 can define a plane for the pair's respective support frame 81, 82, 83. The support members 81, 82, 83 can generally align to define three planes P3, P4, P5 as illustrated in Figure 4C when the end effector 100 is in the unconstrained configuration.

When the loop member 1, 2, 3 is pressed to a surface S as in the sequence of illustrations of Figures 8C and 8D, a majority of the respective length of each segment in each of the respective pairs of parallel segments can become approximately coplanar with a majority of the respective length of each of the other segments in each of the respective pairs of parallel segments. The support members 80 can become aligned with the surface S as the loop members 1, 2, 3 are pressed into the surface S as illustrated in Figure 6B. When the surface S is planar, the parallel segments 81A, 82A, 83A, 81B, 82B, 83B become coplanar with each other along a majority of their respective lengths.

Each support frame 81, 82, 83 can include knuckles to promote conformance of the loop members 1, 2, 3 to the surface S. The knuckles can be spaced uniformly or non-uniformly along the looped path of a respective support member 81, 82, 83. Knuckle features can include thinned out sections of the material of the support members 81, 82, 83. Additionally, or alternatively, the knuckle features can include hinge mechanisms.

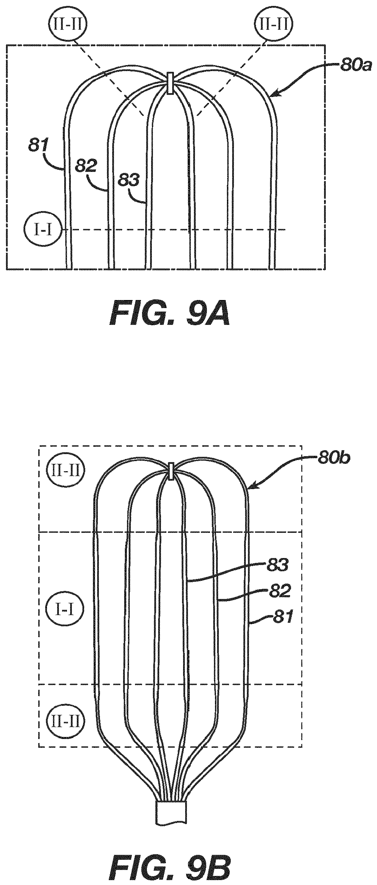

Figures 9A and 9B are illustrations of example support frames that vary in cross section along a respective looped path of each support frame. Each of the support frames 80 can respectively have a cross sectional shape that varies along the individual support frame's looped path, where the cross sectional shape is taken from a cross section orthogonal to the direction of the looped path. Figures 9A and 9B illustrate two different example support frame assemblies 80a, 80b. Each of the example support frame assemblies 80a, 80b having regions I-I with a cross sectional area configured to resist deflection and regions II-II configured to facilitate deflection. The regions I-I configured to resist deflection can have a larger cross sectional area compared to the regions II-II configured to facilitate deflection. Alternatively, the regions I-I configured to resist deflection can be flattened to resist deflection in the direction of long sides of the cross section while having a similar cross section to non-flattened, or less flattened regions II-II. That is, in Figure 9A, the thin section are intended to promote sheath retraction (lower force for the frame to collapse) while in Fig 9B, the distal II-II section is intended still for frame collapsing, but the proximal II-II section (knuckles) are intended to promote deflection with respect to the longitudinal axis L-L.

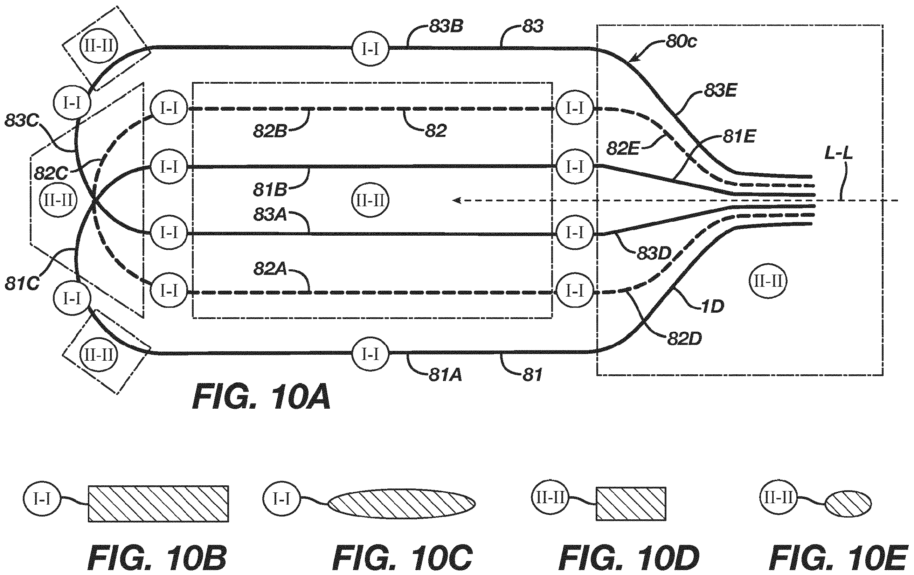

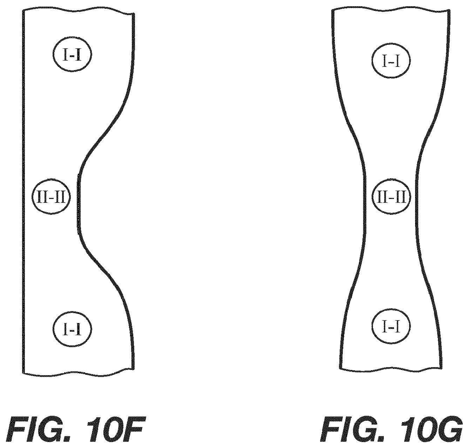

Figure 10A is another illustration of an example support frame assembly 80c of the end effector 100. Figures 10B through 10E are illustrations of cross sectional areas of the example support frame as indicated in Figure 10A. Figures 10F and 10G are illustrations of example transitions schemes between wide and narrow regions.

Figure 10A shows a top view (looking down onto the second plane P2) of another example support frame assembly 80c of an effector 100 (see Figure 4C for orientation). The support frames 81, 82, 83 are annotated with Roman Numerals indicating locations having an approximate cross sectional shape as illustrated in Figures 10B through 10E. Each cross section is taken orthogonal to the respective looped path of each support frame 81, 82, 83. Each support frame 81, 82, 83 varies in cross section along its looped path. By varying the cross sections, each support frame 81, 82, 83 has variable stiffness/flexibility along its looped path.

Figure 10B shows a cross section having a substantially rectangular shape and corresponding to sections of each support frame 81, 82, 83 annotated with Roman Numerals I-I. When unconstrained, the parallel segments 81A, 82A, 83A, 81B, 82B, 83B define a respective plane for each support frame 81, 82, 83. The rectangle is long in the plane of the respective support frame. The rectangle is short in the direction orthogonal to the plane of the respective support frame 81, 82, 83. As understood by a person skilled in the pertinent art, this shape permits greater flexibility along an axis aligned with the short edges of the shape compared to the flexibility along an axis aligned with the long edges of the shape. In some examples, the width of the long side of the rectangle is about 0.012 inches (0.3 millimeters) and the short side of the rectangle is about 0.008 inches (0.2 millimeters).

Figure. 10C shows an alternative cross section having an ovoid or oval-shaped shape and corresponding to sections of each support frame 81, 82, 83 annotated with Roman Numerals I-I. Like the rectangle illustrated in Figure 10B, the oval illustrated in Figure 10C is long in the plane of the respective support frame 81, 82, 83 and short orthogonal to the respective plane, affecting the relatively flexibility in each direction as understood by a person skilled in the pertinent art. In some examples, the width of the long side of the oval is about 0.012 inches (0.3 millimeters) and the short side of the oval is about 0.005 inches (0.13 millimeters).

Figure 10D shows a cross section corresponding to sections of each support frame 81, 82, 83, annotated with Roman Numerals II-II. The cross section illustrated in Figure 10D has a substantially rectangular shape having a width that is shorter in the plane of the respective support frame 81, 82, 83 compared to the width of the rectangular cross section illustrated in Figure 10B. The height of the cross section illustrated in Figure 10D orthogonal to the plane of the support frame 81, 82, 83 can be about equal to the height of the cross section of the rectangle illustrated in Figure 10B. Alternatively, the height of the narrower section II-II can be greater than the height of the wider section I-I. Areas of the respective support frame 81, 82, 83 having a cross section as illustrated in Figure 10D have a higher flexibility in the plane of the respective support frame compared to areas of the respective support frame having a cross section as illustrated in Figure 10B. In some examples, the cross section is approximately square shaped having an edge length of about 0.008 inches (0.2 millimeters).

A support frame 81, 82, 83 including rectangular cross sections illustrated in Figures 10B and 10D can be formed by selecting a sheet having a thickness about equal to the height of the cross sectional shapes illustrated in Figures 10B and 10D and cutting the sheet to the shape of each respective support frame 81, 82, 83 as illustrating in Figure 10A, varying the width of each segment of each support frame 81, 82, 83 to be wider in regions indicated by the Roman Numerals I-I and narrower in regions indicated by Roman Numerals II-II. Alternatively, the support frame 81, 82, 83 can be formed by selecting square or rectangular wire, shaping the wire to form a looped path, and flattening the wire to have regions with wider cross sections I-I and narrower cross sections II-II.

Figure 10E shows an alternative cross section corresponding to sections of each support frame 81, 82, 83 annotated with Roman Numerals II-II. The cross section has an ovoid or oval-shaped shape that is shorter in the plane of the respective support frame 81, 82, 83 compared to the width of the oval cross section illustrated in Figure 10C. The height of the cross section illustrated in Figure 10E orthogonal to the plane of the support frame 81, 82, 83 can be greater than the height of the cross section of the oval illustrated in Figure 10C. Areas of the respective support frame 81, 82, 83 having a cross section as illustrated in Figure 10E have a higher flexibility in the plane of the respective support frame compared to areas of the respective support frame having a cross section as illustrated in Figure 10C. In some examples, the cross section is approximately circular having a diameter of about 0.008 inches (0.2 millimeters).

A support frame 81, 82, 83 including ovoid or oval-shaped cross sections illustrated in Figures 10C and 10E can be formed by selecting a round or oval wire, shaping the wire to form a looped path, and flattening the wire to have regions with wider cross sections I-I and narrower cross sections II-II.

Referring collectively to Figures 10A through 10E, the end effector 100 can include a support frame assembly 80c having some or all of the cross sections illustrated in Figures 10B through 10E in any combination. Further, each support frame 81, 82, 83 can individually include some or all of the cross sections illustrated in Figure 10B through 10E in any combination. The effector 100 can additionally, or alternatively include cross sections not illustrated herein to achieve a difference in flexibility between regions indicated by Roman Numerals I-I and regions indicated by Roman Numerals II-II as understood by a person skilled in the pertinent art according to the teachings herein. Preferably, for the sake of manufacturability, individual support frames 81, 82, 83 can include primarily rectangular cross sectional shapes (e.g. Figures 10B and 10D) or primarily ovoid shapes (e.g. Figures 10C and 10E) as combining rectangular shapes with ovoid shapes in the same support frame 81, 82, 83 can increase cost and/or difficulty in manufacturing.

Figures 10F and 10G are illustrations of possible transition schemes (knuckles) between regions of a support frame of the end effector. As illustrated in Figure 10F, the support frame can transition asymmetrically in width from a wider cross section I-I to a narrower cross section II-II and vice versa. As illustrated in Figure 10G, the support frame can transition symmetrically in width from a wider cross section I-Ito a narrower cross section II-II and vice versa. The support frame can include only asymmetrical transitions in width, only symmetrical transitions in width, or a mix of asymmetrical and symmetrical transitions in width. Such transitions can be applied to any of the example support members illustrated and otherwise described herein.

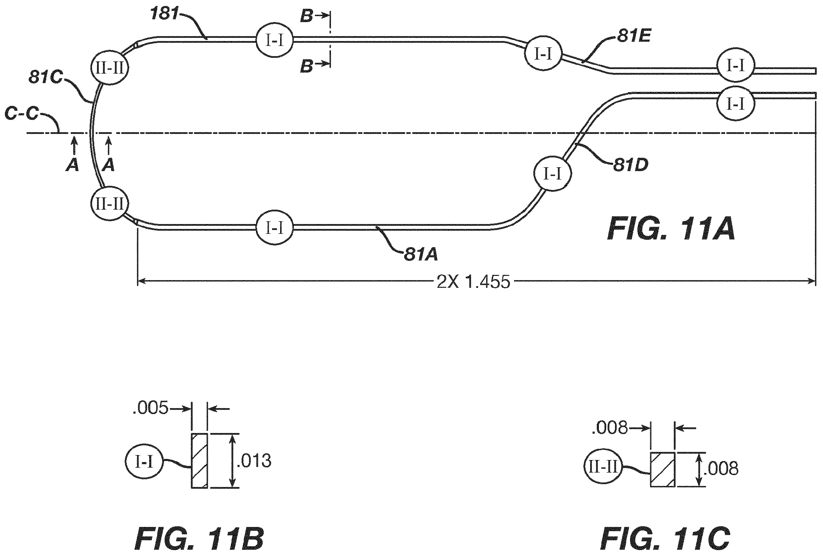

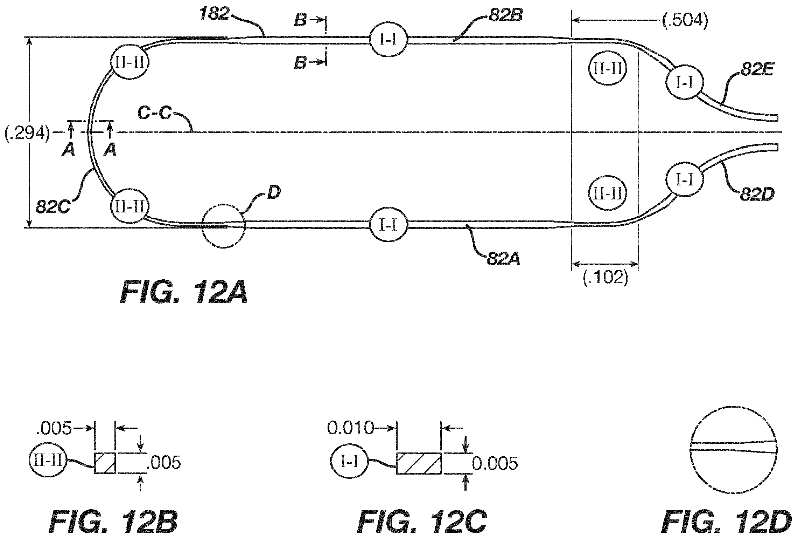

Figure 1 1A is an illustration of an asymmetrical support frame 181 of the end effector 100. Figures 11B and 11C are illustrations of cross sections of the asymmetrical support frame 181 as indicated in Figure 11A. The asymmetrical support frame 181 is another example support frame that can be used in place of the outer support frames 81, 83 illustrated and described elsewhere herein (e.g. in relation to Figures 8A through 8D). Figure 12A is an illustration of a symmetrical support frame 182 of the end effector 100. The symmetrical support frame 182 is another example support frame that can be used in place of the central support frame 82 illustrated and described elsewhere herein (e.g. in relation to Figures 8A through 8D). Figures 12B and 12C are illustrations of cross sections of the symmetrical support frame as indicated in Figure 12A. Figure 12D is an illustration of a detailed section of the symmetrical support frame as indicated in Figure 12A. In some examples, the support frame assembly 80 can include two asymmetrical support frames 181 and a single symmetrical support frame 182.

Figure 11A illustrates that the parallel segments 81A, 81B of the asymmetric support frame 181 can have a wider cross section I-I as illustrated in Figure 11C compared to the narrower cross section II-II of the connecting segment 81C as illustrated in Figure 11B. In some examples, the cross sectional shape of the parallel segments 81A, 81B can be substantially rectangular with a width in the plane of the support frame 181 of about 0.013 inches (0.33 millimeters) and a height orthogonal to the plane of the support frame 181 of about 0.005 inches (0.13 millimeters). In some examples, the cross sectional shape of the connecting segment 81C can be substantially rectangular or square with a width in the plane of the support frame 181 of about 0.008 inches (0.2 millimeters) and a height orthogonal to the plane of the support frame 181 of about 0.008 inches (0.2 millimeters). Further, the proximal segments 81E, 81D can have a cross section I-I of approximately the same dimensions as the parallel segments 81A, 81B.

Figure 12A illustrates that a majority of the length of the parallel segments 82A, 82B of the symmetric support frame 182 can have a wider cross section I-I as illustrated in Figure 12C compared to the narrower cross section II-II of the connecting segment 82C. The parallel segments 82A, 82B can include a tapering transition as illustrated in Figure 12D and indicated in Figure 12A that tapers from the wider width of the wider cross section I-I to the narrower width II-II of the narrower cross section II-II. A distal portion of each parallel segment 82A, 82B distal to the tapering transition can have the narrower cross section I-I. The proximal segments 82E, 83D can have a cross section I-I of approximately the same dimensions as the majority of the length of the parallel segments 82A, 82B. The symmetric support frame 182 can further include a narrower width sections 82F, 82G that respectively include a proximal portion of a respective parallel segment 82A, 82B and a distal portion of a respective proximal segment 82D, 82E. These narrower width sections 82F, 82G can have a cross section II-II as illustrated in Figure 12B. These narrower width sections 82F, 82G can have a length of approximately 0.102 inches (2.6 millimeters). The symmetric support frame 82 can have a width of approximately 0.294 inches (7.5 millimeters) as measured between outer edges of the parallel segments 82A, 82B in the plane of the support frame 82 as illustrated.

As illustrated in Figure 12B, in some examples, the narrower cross sectional regions II-II can have a cross sectional shape that is substantially rectangular or square with a width in the plane of the support frame 182 of about 0.005 inches (0.13 millimeters) and a height orthogonal to the plane of the support frame 182 of about 0.005 inches (0.13 millimeters).

As illustrated in Figure 12C, in some examples, the wider cross sectional regions I-I can have a substantially rectangular cross sectional shape with a width in the plane of the support frame 182 of about 0.01 inches (0.25 millimeters) and a height orthogonal to the plane of the support frame 182 of about 0.005 inches (0.13 millimeters).

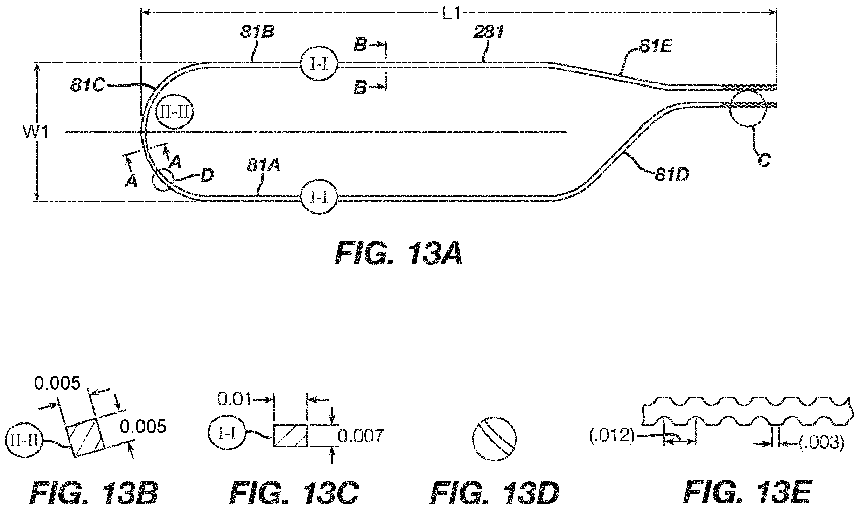

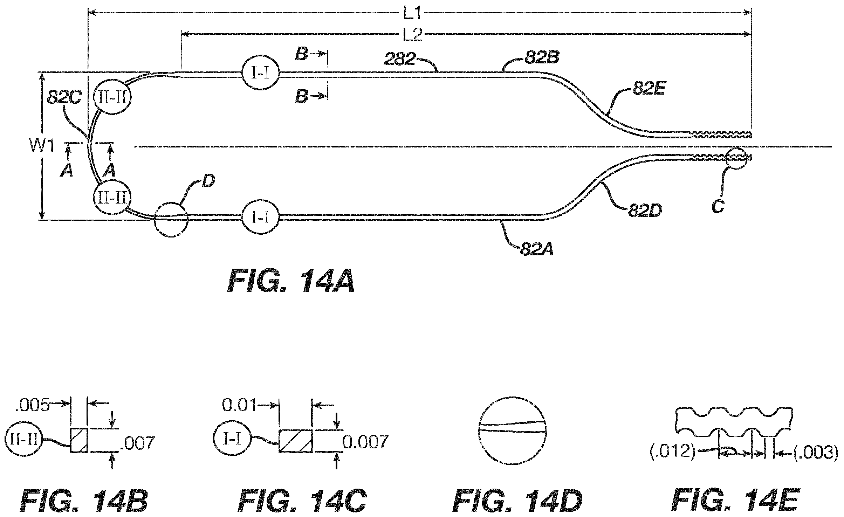

Figure 13A is an illustration of an asymmetrical support frame 281 of the end effector 100. Figures 13B and 13C are illustrations of cross sections of the asymmetrical support frame 281 as indicated in Figure 13A. The asymmetrical support frame 281 is another example support frame that can be used in place of the outer support frames 81, 83 illustrated and described elsewhere herein (e.g. in relation to Figures 8A through 8D). Figures 13D and 13E are illustrations of detailed sections of the asymmetrical support frame 281 as indicated in Figure 13A. Figure 14A is an illustration of a symmetrical support frame 282 of the end effector 100. The symmetrical support frame 282 is another example support frame that can be used in place of the central support frame 82 illustrated and described elsewhere herein (e.g. in relation to Figures 8A through 8D). Figures 14B and 14C are illustrations of cross sections of the symmetrical support frame as indicated in Figure 14A. Figures 14D and 14E are illustrations of a detailed section of the symmetrical support frame as indicated in Figure 14A. In some examples, the support frame assembly 80 can include two asymmetrical support frames 281 and a single symmetrical support frame 282.

Figure 13A illustrates that the parallel segments 81A, 81B of the asymmetric support frame 281 can have a wider cross section I-I as illustrated in Figure 13B compared to the narrower cross section II-II of the connecting segment 81C. In some examples, the cross sectional shape of the parallel segments 81A, 81B can be substantially rectangular with a width in the plane of the support frame 281 of about 0.01 inches (0.25 millimeters) and a height orthogonal to the plane of the support frame 281 of about 0.007 inches (0.18 millimeters). In some examples, the cross sectional shape of the connecting segment 81C, at least in the area indicated by section A-A in Figure 13A, can be substantially square with a width in the plane of the support frame 281 less than the width of the cross section of the parallel segments 81B, 81A. The cross section I-I can have a width of about 0.005 to about 0.007 inches (about 0.13 to 0.18 millimeters). The cross section I-I can have a height of about 0.007 inches (about 0.18 millimeters). Further, the proximal segments 81E, 81D can have a cross section I-I of approximately the same dimensions as the parallel segments 81A, 81B. A length L1 of the frame 81B as measured from a distal end to the proximal end (indicated as "y" in Fig. 13A) can be approximately 1.4 inches (or 36 mm) and a width W1 as measured from frame 81A to frame 81B is approximately 0.29 inches (or 7.4 mm).

Figure 13D illustrates transition, on the connector segment 81C between the wider cross section I-I illustrated in Figure 13D and the narrower cross section II-II illustrated in Figure 13C.

Figure 13E illustrates a serrated segments of the support frame 281 shaped to secure the two ends of the loop member to the distal portion of the shaft 9 or connector tubing 46.

Figure 14A illustrates that a majority of the length of the parallel segments 82A, 82B of the symmetric support frame 282 can have a wider cross section I-I as illustrated in Figure 14C compared to the narrower cross section II-II of the connecting segment 82C as illustrated in Figure 14B. The parallel segments 82A, 82B can include a tapering transition as illustrated in Figure 14D and indicated in Figure 14A that tapers from the wider width of the wider cross section I-I to the narrower width II-II of the narrower cross section II-II. Figure 14E illustrates a serrated segments of the support frame 282 shaped to secure the two ends of the loop member to the distal portion of the shaft. We have devised a configuration of the support frame in Fig. 14A (as well as Fig. 13A) such that an aspect ratio of the length L1 to width W1 (i.e., L1/W1) may be from 4 to 5. In the preferred embodiments, the aspect ratio (L1/W1) of the frame support of Figs. 13A and 14A is one of 4.5 or 4.75.

As illustrated in Figure 14B, in some examples, the narrower cross sectional regions II-II can have a cross sectional shape that is substantially rectangular with a width in the plane of the support frame 282 of about 0.005 inches (0.13 millimeters) and a height orthogonal to the plane of the support frame 282 of about 0.007 inches (0.18 millimeters).

As illustrated in Figure 14C, in some examples, the wider cross sectional regions I-I can have a substantially rectangular cross sectional shape with a width in the plane of the support frame 282 of about 0.01 inches (0.25 millimeters) and a height orthogonal to the plane of the support frame 282 of about 0.005 inches (0.13 millimeters).

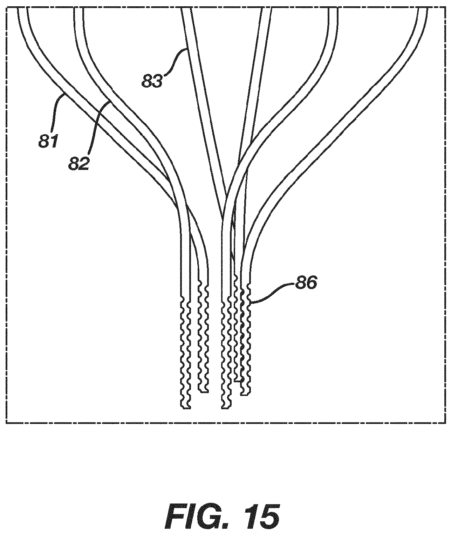

Figure 15 is an illustration of crimped or serrated features 86 at ends of the support frames. The crimped or serrated features 86 can facilitate engagement of the support frames 81, 82, 83 to the distal end 14A of the shaft 9 (e.g. within the connector tubing 46). In particular, the serrations 86 of each support frame can be molded together with the connector tubing 46 or affixed (e.g., adhesive or epoxy) with the connector tubing 46. To ensure that each loop frame is structurally rigid where the loop comes out of the tubing 46, each serration 86 of each support frame are affixed directly to the tubing 46 and one serration 86 does not engage or mate with another serration 86 from another support member. This ensures that forces transmitted to frame 81 from the loop distal end are not transmitted to itself via the serrations or to other frames 82 and 83 due to the serrations 86 being independently affixed to the tubing 46 instead of other serrations 86.

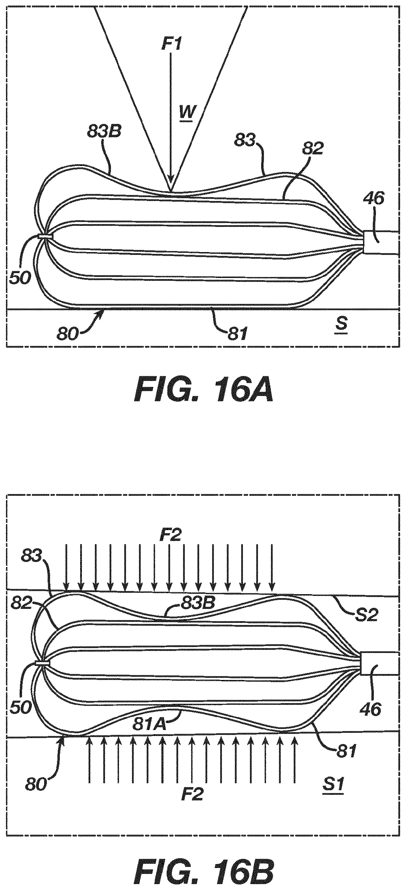

Figures 16A through 16D are illustrations of deformation of support frame assemblies as a result of application of various forces. Each support frame assembly 80 includes a first support frame 81, a second support frame 82, and a third support frame 83. The first and third support frames 81, 83 are outer support frames surrounding the second central support frame 82. The support frame assembly 80 includes a mechanical linkage 50 joining the first, second, and third support frames 81, 82, 83 at the distal vertex.

Figure 16A illustrates the first support frame 81 pressed into a planar surface S as an apex of a wedge W is pressed with a force F1 into the third support frame 83. The support frame assembly 80 is aligned approximately orthogonal to the planar surface S. The force F1 is applied approximately orthogonal to, and in a direction toward the planar surface S. The force F1 is applied centrally along the length of the outer parallel segment 83B of the third support frame 83. The outer parallel segment 83B of the third support frame bows toward the surface S as a result of the force F 1. The support frame assembly 80 can be configured to resist spine-to-spine contact when the force F1 is applied. In Figure 16A, the outer parallel segment 83B of the third support frame is bowed to contact the second support frame 82. The force F1 sufficient to move the outer parallel segment 83B of the third support frame 83 into contact with the second support frame 82 can be a metric used to compare variations in support frame assembly design (e.g. with differing support frame cross section design) where a higher force F1 indicates a more favorable result for this test.

Figure 16B illustrates the support frame assembly 80 compressed between two planar surfaces S1, S2 with a force F2. The planar surfaces S1, S2 are aligned approximately parallel to each other. The support frame assembly 80 is aligned approximately orthogonal to the planar surfaces S1, S2. An outer parallel segment 81A of the first support frame 81 is pressed by the first planar surface S1. The outer parallel segment 83B of the third support frame 83 is pressed by the second planar surface S2. As a result of the compression by the force F2 between the planar surfaces S1, S2, the outer parallel segments 81A, 83B bow toward the opposite planar surface S1, S2. The support frame assembly 80 can be configured to resist spine-to-spine contact when the force F2 is applied. The force F2 sufficient to move the outer parallel segment 83B of the third support frame 83 or the outer parallel segment 81A of the first support frame 81 into contact with the second support frame 82 can be a metric used to compare variations in support frame assembly design (e.g. with differing support frame cross section design) where a higher force F2 indicates a more favorable result for this test. In the preferred embodiments, F1 or F2 is approximately 10 gram-force or greater. The range of suitable force is about 10 gram-force to 50 gram-force for F1 and 10 gram-force to 70 gram-force.

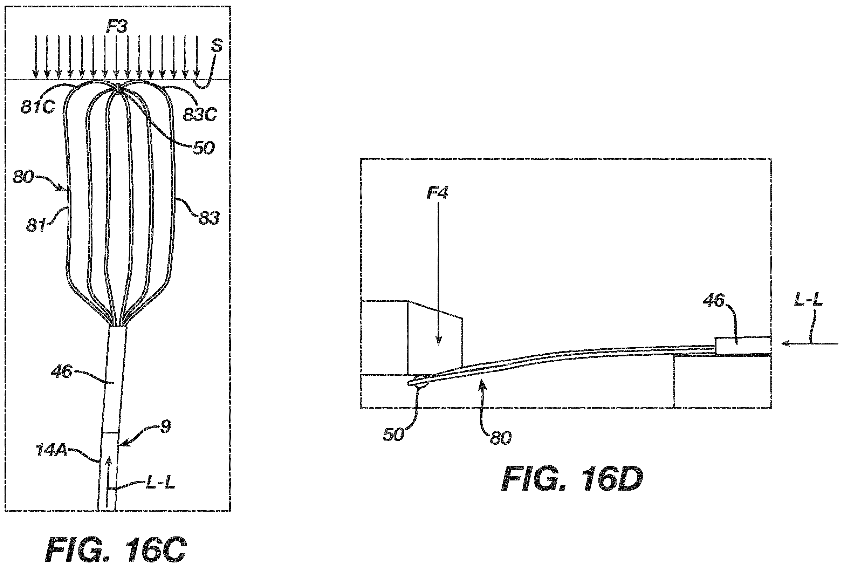

Figure 16C illustrates the support frame assembly 80 compressed by a planar surface S applying a force F3 to a distal end of the support frame assembly 80. The distal portion 14A of the shaft 9 is held at a position in the proximal direction of the connector tubing 46 such that the longitudinal axis L-L defined by the shaft 9 is approximately orthogonal to the surface S. The force F3 is applied to the connector segments 81C, 83C of the first and third support frames 81, 83. As a result of the compression by the force F3 in the direction of the longitudinal axis L-L, the support frame assembly 80 and connector tubing 46 deflect out of alignment with the longitudinal axis L-L. This is to ensure the distal section 14A of shaft 9 and/or end effector are sufficiently flexible so that the end effector and/or distal section of the shaft buckles at a sufficiently low force when applied to a heart wall to inhibit the end effector from puncturing the heart wall.

Figure 16D illustrates the support frame assembly 80 deflected by a force F4 applied approximately orthogonal to the support frame assembly near the mechanical linkage 50 at the distal vertex. The distal portion 14A of the shaft 9 is held near the connector tubing 46. As a result of the force F4, the support frame 80 is deflected out of alignment with the longitudinal axis L-L. In some use cases, it can be desired to have a greater or lesser deflection as a result of Force F4 depending on the geometry of a treatment area and/or preferences of a physician regarding tactile feedback.

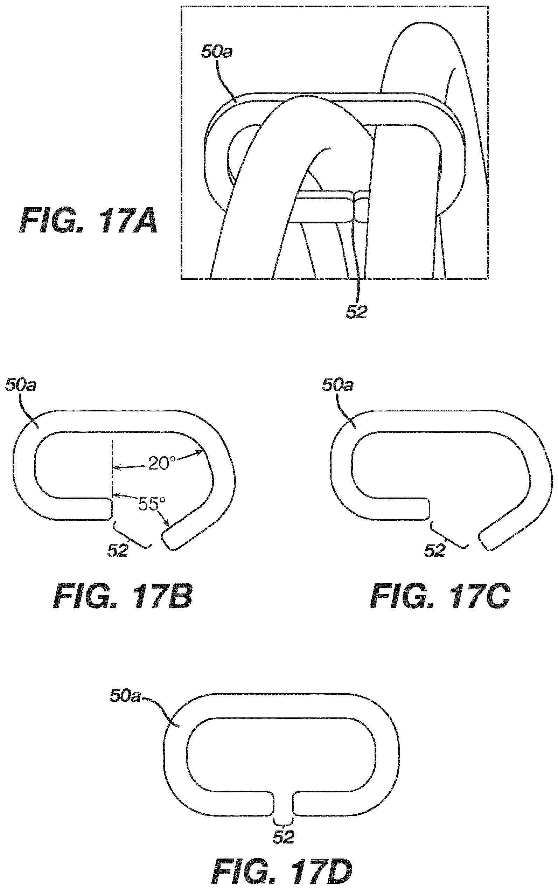

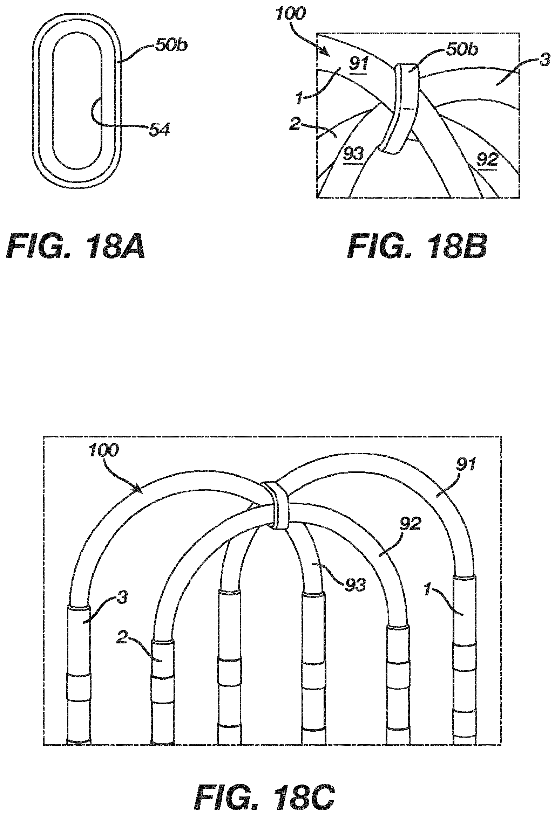

Figures 17A through 17D are illustrations of an example rectangular or ovular mechanical linkage 50a having a closable single opening and used for joining the loop members according to aspects of the present invention. Alternative mechanical linkages 50b-h are illustrated in Figures 18A through 22C. The linkages 50a-h can be used to join loop members (or other electrode carrying structures) of catheter-based devices similar to how the loop members 1, 2, 3 are joined at the distal vertex as described and illustrated herein. The mechanical linkage can serve to inhibit electrodes 37 on the spines 1A, 2A, 3A, 1B, 2B, 3B of the end effector 100 from coming into contact with each other. Loop members 1, 2, 3 are spatially affixed relative to each other by the connector tubing 46 near the proximal end of the end effector 100. Without the mechanical linkage, the distal ends of the respective loop members 1, 2, 3 are free to move in relation to each other when acted on by a force such as the forces F1, F2, F3, F4 illustrated in Figures 16A through 16D. The mechanical linkage is sized, shaped, and otherwise configured to allow the end effector 100 to be collapsed for delivery through a catheter or guiding sheath to a treatment site. Ease of assembly of the end effector 100 is also a design consideration for the mechanical linkage.

Figure 17A illustrates the mechanical linkage 50a joining three loop members 1, 2, 3. The mechanical linkage 50a includes a seam or gap 52. Figures 17B and 17C illustrate the mechanical linkage 50a as manufactured. The linkage 50a is initially formed as an open clip. A stiff material, preferably metal, is formed or cut into a shape resembling an open paper clip. The individual loop members 1, 2, 3 can be constructed prior to being joined by the mechanical linkage 50a. In some example methods of construction, the end effector 100 can be entirely contracted and affixed to the shaft 9 before the linkage 50a is affixed to the loop members 1, 2, 3. The linkage 50a can include an opening 52 sized to allow loop members 1, 2, 3, one at a time, to be inserted into the linkage 50a. The opening 52 as manufactured can be larger than respective diameters of the loop members 1, 2, 3 near the distal vertex. Once the loop members 1, 2, 3 are inserted into the linkage 50a, the opening 52 can be collapsed as illustrated in Figure 17D. The opening 52 can be collapsed by crimping a free open end of the linkage 50a until it lines up with the other open end of the linkage 50a. When the opening is collapsed, a short side of the linkage 50a can be moved through an angle of about 20° and the free open end on a long side of the linkage 50a can be moved through an angle of about 59° as illustrated in Figure 17B.

The mechanical linkage 50a illustrated in Figures 17A through 17D is symmetrical. Alternatively, the mechanical linkage 50a can be asymmetrical to promote consistent collapsing geometry of the end effector for delivery through a catheter.

Figures 18A through 18C are illustrations of an example rectangular or ovular mechanical linkage 50b having a single opening and used for joining the loop members 1, 2, 3. The example mechanical linkage 50b has four contiguous sides. Compared to the example mechanical linkage 50a illustrated in Figures 17A through 17D, the example mechanical linkage 50b in Figures 18A through 18C lacks a gap or seam 52. During assembly, loop members 1, 2, 3 can be fed through the opening 54 of the linkage 50b before the ends of the loop members 1, 2, 3 are affixed to the shaft 9.

The mechanical linkage 50b illustrated in Figures 18A through 18C is symmetrical. Alternatively, one side of the ring can be wider than the other to promote the loop members 1, 2, 3 to fold to a particular side when the end effector 100 is collapsed for delivery through a catheter or guiding sheath.

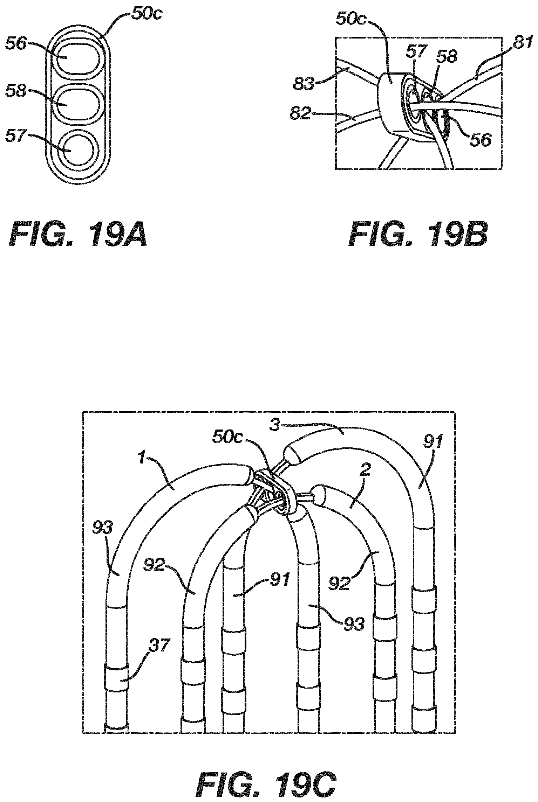

Figures 19A through 19C are illustrations of an example rectangular or ovular mechanical linkage 50c having three openings 56, 57, 58 and used for joining the loop members 1, 2, 3. Each opening 56, 57, 58 can be shaped and otherwise configured to receive a loop member 1, 2, 3.

The linkage 50c can include a circular opening 57 to receive the central, symmetric loop member 2. The central loop member 2 can be approximately orthogonal to the linkage 50c at the distal vertex, allowing for the respective opening 57 of the linkage 50c to be circular. The linkage 50c can include two oblong openings 58, 56 each respectively shaped to receive a respective outer, asymmetrical loop member 1, 3. The outer loop members 1, 3 can pass through the linkage 50c at a non-orthogonal angle. The oblong shape of the corresponding linkage openings 56, 58 can be elongated to account for non-orthogonal trajectory of the outer loop members 1, 3 through the linkage 50c.

Each of the loop members 1, 2, 3 can include a respective tubular housing 91, 92, 93 covering a majority of the support frame 81, 82, 83 for that loop member 1, 2, 3. To reduce the separation of the support frames 81, 82, 83 at the distal vertex, the loop members 1, 2, 3 need not include tubular housing 91, 92, 93 near the distal vertex. The openings 56, 57, 58 of the linkage 50c can be sized to allow the support frame 81, 82, 83 of the loop members to pass through, but need not be sized to allow the tubular housings 91, 92, 93 to pass through. During assembly, the support frames 81, 82, 83 can be positioned through the openings 56, 57, 58 of the linkage 50c before the tubular housings 91, 92, 93 are added to the loop members 1, 2, 3.

Alternatively, the openings 56, 57, 58 can be sized to allow the tubular housings 91, 92, 93 of the loop members 1, 2, 3 to pass therethrough to allow for a design where some or all of the tubular housings 91, 92, 93 cross the distal vertex and/or to allow for an assembly process where the tubular housings 91, 92, 93 are affixed to the loop members 1, 2, 3 before the mechanical linkage 50c.

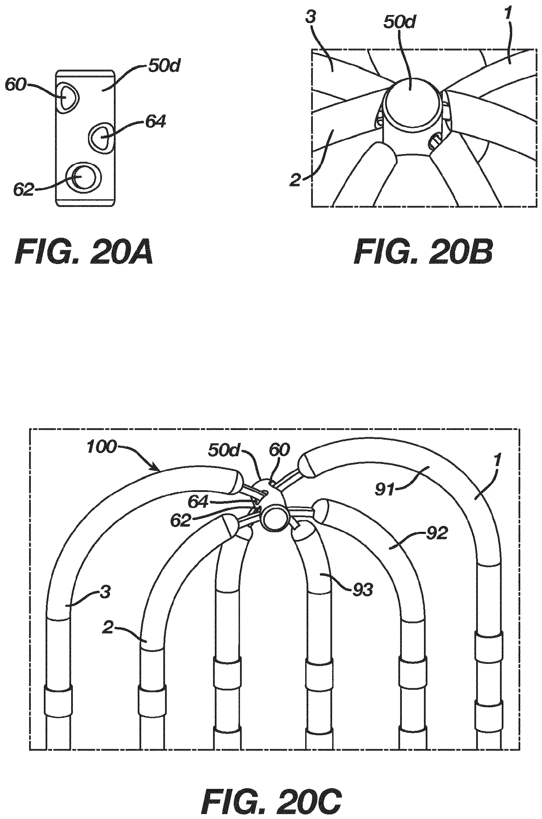

Figures 20A through 20C are illustrations of an example cylindrical mechanical linkage 50d having three passageways 60, 62, 64 and used for joining support members 81, 82, 83 of the loop members 1, 2, 3. The openings 60, 62, 64 of the linkage 50d can be sized to allow the support frame 81, 82, 83 of the loop members to pass through, but need not be sized to allow the tubular housings 91, 92, 93 to pass through. During assembly, the support frames 81, 82, 83 can be positioned through the openings 60, 62, 64 of the linkage 50d before the tubular housings 91, 92, 93 are added to the loop members 1, 2, 3.

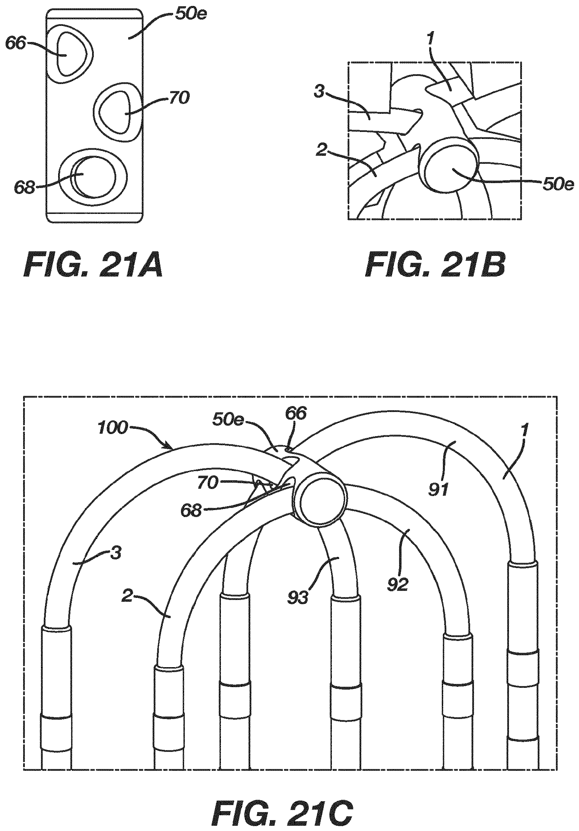

Figures 21A through 21C are illustrations of an example cylindrical mechanical linkage 50e having three passageways 66, 68, 70 and used for joining the loop members 1, 2, 3 with tubular housings 91, 92, 93 over the support members 81, 82, 83. In some examples, the passageways 66, 68, 70 can be sized to allow for an assembly process where the tubular housings 91, 92, 93 are affixed to the loop members 1, 2, 3 before the mechanical linkage 50e.

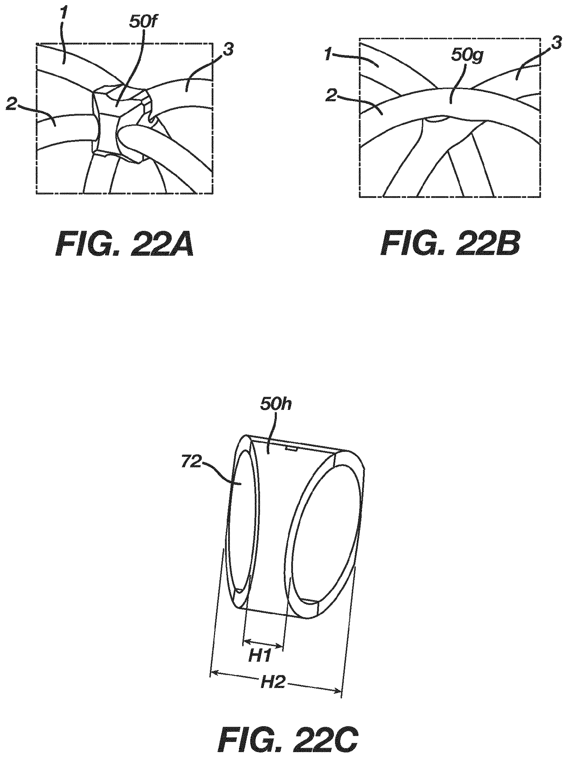

Figures 22A through 22C are illustrations of additional example mechanical linkages. Figure 22A illustrates a mechanical linkage 50f including polymer shaped to closely conform to the dimensions of the loop members 1, 2, 3. The linkage 50f can include an adhesive. The linkage 50f can be applied by hand, over molded, or by other means as understood by a person skilled in the pertinent art. Figures 22B illustrates a mechanical linkage 50g including an adhesive. Figure 22C illustrates a tapered ring linkage 50h having an annular opening 72 through which the three loop members 1, 2, 3 can extend and a height that tapers from a larger dimension H2 to a smaller dimension H1 across the diameter of the opening 72. The link 50h has a portion on one side that is narrower on one side (H1) as compared to the portion H2 on the other side. The narrowed portion H1 encourages the loops 1, 2 and 3 to bend toward the narrowed side when retracting into the sheath. This can be used to reduce forces required for sheath retraction.

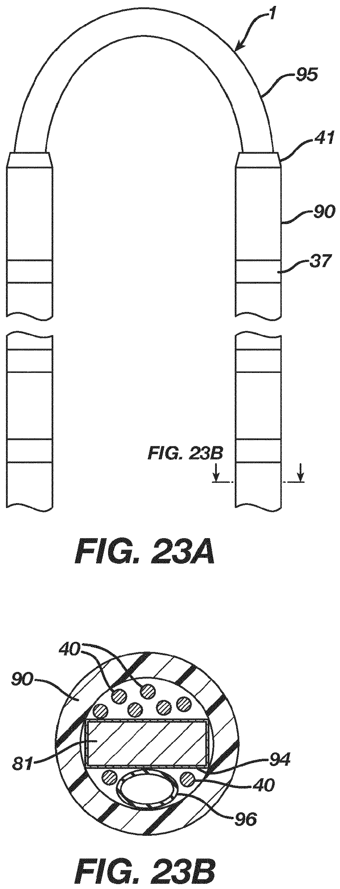

Figures 23A and 23B are illustrations of an example loop member 1 having an inner housing 94 and outer housing 90 surrounding the support frame 81. The illustrated loop member 1 can be used in place of loop members 1, 2, 3 illustrated and otherwise disclosed herein according to the teachings herein. In particular, it can be advantageous to use the illustrated loop member in place of loop members 1, 2, 3 illustrated in Figures 19C and 20C. The inner housing 94 and outer housing 90 can collectively function as the tubular housing 91, 92, 93 illustrated elsewhere herein.

The outer housing 90 can include polymeric tube (e.g. thermoplastic polyurethane) having a single lumen sized to house the support frame 80 and wires 40 that provide electrical connections to the end effector electrodes 37. The lumen of the outer housing 90 can also be sized to house an irrigation tube 96 having an irrigation lumen therethrough. In this configuration, the support frame is surrounded by a sleeve to isolate its edges from damaging the conductors. The support frame, sleeve and conductors are in one lumen, shown in Fig 23B (whereby irrigation is optional). A small diameter tube 95 in Fig. 23A is a separate piece that is bonded to the larger tube, with the purpose of reducing the outer diameter when passing through the mechanical linkage.

The inner housing 94 (Fig. 23B) can be shaped, sized, and otherwise configured to closely surround the support frame 81. The inner housing 94 can include a polymeric material, for example a shrink sleeve applied during a reflow process. Because neither the wires 40 nor the irrigation tube 96 need extend beyond the spines of the loop member, the outer housing 90 need not cover the connector segment of the loop member. The inner housing 94 can be dimensioned to allow less separation between the loop members 1, 2, 3 near the distal vertex compared to a loop member having a tubular housing dimensioned similar to the outer housing 90 crossing, or extending near, the distal vertex. Further, the distal end of the end effector 100 can collapse to a smaller dimension compared to an end effector having a tubular housing dimensioned similar to the outer housing 90 crossing, or extending near, the distal vertex.

Within the outer housing 90, the inner housing 94 can be bonded to the outer housing 90. The bond between the inner housing 94 and outer housing 90 can inhibit fluid leakage. The bond between the inner housing 94 and outer housing 90 can promote conformity of the shape of the outer housing 90 to that of the support frame 81 inhibiting shifting of the support frame 81 within the outer housing 90 when the end effector 100 is deformed from its unconstrained configuration.

At the distal ends of the outer housing 90, the loop member 1 can include a joint 41 configured to inhibit fluid ingress into the outer housing 90. The joint 41 can be bonded to the outer housing 90 and inner housing 94.

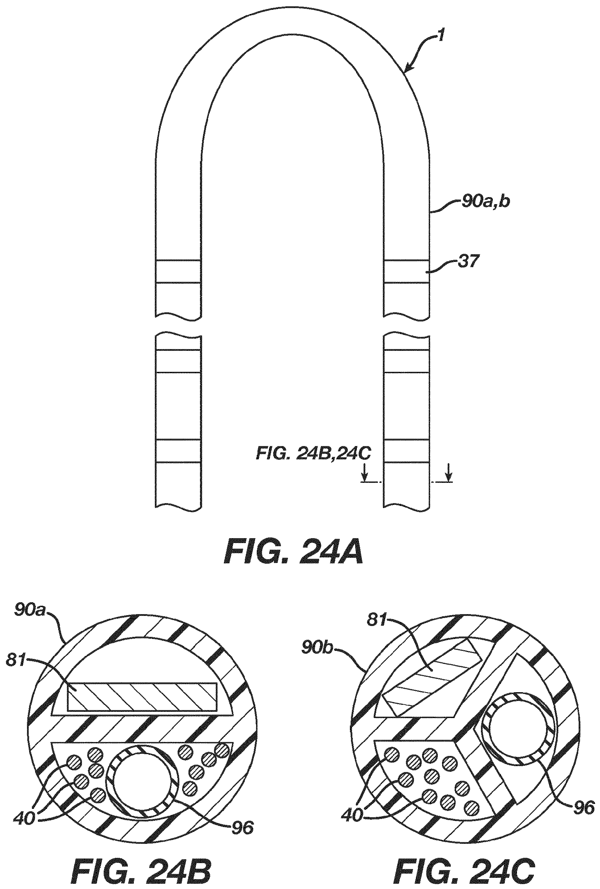

Figures 24A through 24C are illustrations of an alternative example loop member having a tubular housing 90a, 90b having two lumens 90a or three lumens 90b therethrough in which the support frame 81, conductive wires 40, and an irrigation tube 96 are positioned. Regardless of whether the tubular housing 90a, 90b has two or three lumens, the loop member can have an outer appearance similar to as illustrated in Figure 24A. Figure 24B illustrates a cross section of the loop member having a tubular housing 90a with two lumens. The design purpose of the embodiment in Fig. 24B is to isolate the support member from damaging the conductors or irrigation lines. Figure 24C illustrates a cross section of the loop member having a tubular housing 90b with three lumens. The cross sections illustrated in Figures 24B and 24C are viewed along a spine of the loop member as indicated in Figure 24A.

Figure 24B illustrates the support frame 81 positioned in one lumen of the dual lumen tubular housing 90a and the wires 40 and irrigation tube 96 positioned in the other lumen of the dual lumen tubular housing 90b. Separation of the support frame 81 from the wires 40 and irrigation tube 96 can inhibit edges of the support frame 81 from abating insulation the wires 40 or walls of the irrigation tube 96. The dual lumen tubular housing 90b can be used in place of tubular housings 91, 92, 93 illustrated elsewhere herein.

Figure 24C illustrates the support frame 81 positioned in a first lumen of the tri lumen tubular housing 90b, wires 40 in a second lumen of the tri lumen tubular housing 90b, and an irrigation tube 96 in a third lumen of the tri lumen tubular housing 90b. The tri lumen tubular housing 90c can be used in place of tubular housings 91, 92, 93 illustrated elsewhere herein. The purpose of this design in Fig. 24C is to isolate the irrigation line 96 from contact with the other materials. As an alternative to the illustration of Fig. 24C, the loop member 1 need not include an irrigation tube 96, and the lumen of the tubular housing 90b in which the irrigation tube 96 is illustrated can be used direction for irrigation.

Spine covers 90, 90a, 90b can be preferably 3 French to 2 French. In some examples, the spine cover 90 illustrated in Figures 23A and 23B, compared to the spine covers 90a, 90b illustrated in Figures 24A through 24B, can have more usable interior space for the same French sizing, allowing for a larger support frame, larger irrigation tube 96, and/or increased volume of wires 40.

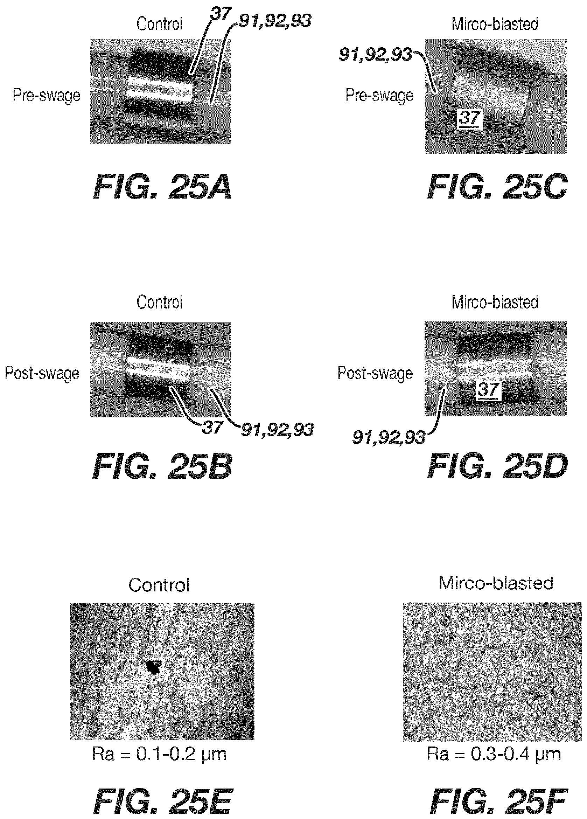

Figures 25A through 25F are illustration of example end effector electrodes 37 with varying degrees of surface roughness. Contact resistance between an electrode and tissue is inversely proportionate to the surface area of the electrode in contact with the tissue. In other words, electrical energy is transferred more efficiently from the electrode to the tissue and vice versa when contact area between the electrode and tissue is increased. For diagnostic measurements, more efficient electrical energy transfer from tissue to electrode results in clearer, less noisy, more accurate electrical signals (sensor measurements). Dimensions of the footprint, or perimeter, of the electrode is limited by the geometry of the vasculature through which the end effector 100 travels to reach a treatment site, the geometry of the treatment site, and the geometry of other components of the end effector 100. Micro-roughness on the electrode surface increases effective surface area of the electrode, thereby reducing contact resistance when the electrode surface is pressed to tissue, without increasing the footprint of the electrode. However, increasing surface roughness can also promote thrombus formation on the electrode which can lead to complications during treatment.

Figure 25A illustrates a control electrode 37 with an untreated surface. Figure 25B illustrates an electrode 37 compacted from a swaging process. The compacted electrode is compressed to have an outer circumference that approximates the outer circumference of the tubular housing 91, 92, 93. Figure 25C illustrates a micro-blasted electrode 37 that is not swaged. Micro-blasting introduces surface roughness on a micrometer scale. The surface can alternatively be roughed by other suitable process such as chemical etching, sputtering, and/or other deposition method. Figure 25D illustrates a micro-blasted and swaged electrode 37.

The combination of roughening (e.g. micro-blasting) and swaging the electrode surface creates a controlled, shallow surface roughness. Swaging reduces the ring diameter of the electrode 37 and can also flatten some vertical features created from roughening, resulting in an electrode with a higher surface area and relatively flattened outer surface. The higher surface area can be effective to reduce contact resistance between an electrode and tissue.

Surface roughness can be characterized by roughness parameter *Ra* representing an arithmetical mean deviation of a profile of the surface. As illustrated in Figure 25F, in some examples, some or all of the electrodes 37 of the end effector 100 can have a surface roughness parameter *Ra* measuring from about 0.3 micrometers to about 0.4 micrometers. This surface roughness can be effective to increase surface area of the electrodes 37 to decrease contact resistance to tissue while not significantly promoting thrombus formation. In comparison, as illustrated in Figure 25E, the untreated surface can have a surface roughness parameter *Ra* measuring from about 0.1 micrometers to about 0.2 micrometers.

During manipulation of the apparatus (e.g. pressing to a surface and/or deflection of the intermediate section 14) wires 40 carrying electrical signals of the end effector electrodes 37 can shift and vibrate. This vibration can cause noise in the electrical signals carried by the wires 40. In some examples, wires 40 can be twisted, as a bundle, within the end effector 100 and/or within the shaft 9 to inhibit movement and vibration of the wires 40. Additionally, or alternatively, other strategies for bundling wires 40 to inhibit wire movement can include shrink sleeving the wires 40, using adhesive to join the wires 40, and/or braiding the wires 40. Additionally, or alternatively, pairs of the wires 40 connected to a bipolar pair of electrodes 37 can be twisted within a respective spine 1A, 2A, 3A, 1B, 2B, 3B to promote electromagnetic compatibility between each twisted pair, thereby reducing electrical noise in electrical signals carried by the respective twisted pairs.
1. An apparatus comprising:
   an elongated shaft comprising a proximal portion and a distal portion, the elongated shaft configured to be manipulated at the proximal portion to position the distal portion into a heart of a patient, the elongated shaft defining a longitudinal axis of the apparatus; and
   an end effector disposed proximate the distal portion of the elongated shaft, the end effector comprising three loop members overlapping at a common distal vertex along the longitudinal axis, each of the three loop members comprising respective pair of ends affixed to the distal portion of the elongated shaft, the end effector configured to expand to an unconstrained configuration when unconstrained to define three different planes for the respective three loop members, so that when distal end of the elongated shaft is deflected at an angle relative to the longitudinal axis and the loop members are positioned against a planar surface, a majority of a length of each of the three loop members becomes contiguous to the planar surface in a flattened configuration.
2. The apparatus of embodiment 1,
   each of the three loop members respectively comprising a support frame extending through a respective loop member of the three loop members and affixed to the distal portion of the elongated shaft at each end of the respective pair of ends of the respective loop member,
   each of the respective support frames defining a respective looped path of the respective loop member when the end effector is in the unconstrained configuration, and
   each of the respective support frames comprising a respective cross sectional shape orthogonal to the respective looped path, each of the respective cross sectional shapes varying along the respective looped path.
3. The apparatus of embodiment 2,
   each of the respective support frames comprising:
   a respective pair of parallel segments, each segment in the respective pair of parallel segments comprising a respective length,
   a majority of the respective length of each segment in each of the respective pairs of parallel segments being approximately coplanar with a majority of the respective length of each of the other segments in each of the respective pairs of parallel segments when the end effector is in the flattened configuration, and
   the majority of the respective lengths the segments of at least one of the respective pairs of parallel segments being non-coplanar with the majority of the respective length of at least one segment of the other respective pairs of parallel segments when the end effector is in the unconstrained configuration.
4. The apparatus of embodiment 1, further comprising:
   a mechanical linkage binding the three loop members at the distal vertex, the mechanical linkage comprising at least one of:
   a rectangular or ovular shape comprising an opening through which the three loop members extend and a side comprising a seam,
   a rectangular or ovular shape comprising an opening through which the three loop members extend and four contiguous sides,
   a rectangular or ovular shape comprising three openings each comprising a respective loop member of the three loop members extending therethrough,
   a cylindrical shape comprising three passageways therethrough, the three passageways each comprising a respective loop member of the three loop members extending therethrough, and
   a tapered ring comprising an annular opening through which the three loop members extend and a tapered height extending across a diameter of annular opening.
5. The apparatus of embodiment 1, further comprising:
   at least one pull wire extending through the elongated shaft and attached to the distal portion of the elongated shaft so that when the pull wire is retracted toward the proximal portion relative to the elongated shaft, the distal portion and the end effector are bent at an angle with respect to the longitudinal axis.
6. An apparatus for a mapping catheter comprising:
   a tubular member extending along a longitudinal axis;
   a first loop member comprising two spine members extending from the tubular member and connected to an arcuate member, the first loop member arrayed on a first plane;
   a second loop member comprising two spine members extending from the tubular member and connected to an arcuate member, the second loop member arrayed on a second plane that intersects with the first plane;
   a third loop member comprising two spine members extending from the tubular member and connected to an arcuate member, the third loop member arrayed on a third plane that intersects with the first plane and the second plane so that majority of the length each of the three loop members is non-coplanar with the majority of the length of at least one of the other three loop members in an unconstrained configuration; and
   each of the first, second and third loop members including a plurality of electrodes disposed thereon each loop member.
7. The apparatus of embodiment 6,
   each of the three loop members respectively comprising a support frame extending through a respective loop member of the three loop members and affixed to a distal portion of the tubular member,
   each of the respective support frames defining a respective looped path of the respective loop member, and
   each of the respective support frames comprising a respective cross sectional shape orthogonal to the respective looped path, each of the respective cross sectional shapes varying along the respective looped path.
8. The apparatus of embodiment 2 or embodiment 7,
   the three loop members each comprising:
   an inner tubular housing surrounding at least a portion of the respective support frame;
   an outer tubular housing surrounding at least a portion of the inner tubular housing and bonded to the inner tubular housing; and
   a plurality of electrical conductors disposed at least partially within the outer tubular housing and outside of the inner tubular housing.
9. The apparatus of embodiment 7,
   the three loop members movable to a flattened configuration when the loop members are positioned against a planar surface, a majority of a length of each of the three loop members being contiguous to the planar surface in the flattened configuration, and
   each of the respective support frames comprising:
      a respective pair of parallel segments, each segment in the respective pair of parallel segments comprising a respective length,
      a majority of the respective length of each segment in each of the respective pairs of parallel segments being approximately coplanar with a majority of the respective length of each of the other segments in each of the respective pairs of parallel segments when the three loop members are in the flattened configuration, and
      the majority of the respective lengths the segments of at least one of the respective pairs of parallel segments being non-coplanar with the majority of the respective length of at least one segment of the other respective pairs of parallel segments when the three loop members are in the unconstrained configuration.
10. The apparatus of embodiment 6, further comprising:
   a mechanical linkage binding the arcuate members of the three loop members, the mechanical linkage comprising at least one of:
   a rectangular or ovular shape comprising an opening through which the three loop members extend and a side comprising a seam,
   a rectangular or ovular shape comprising an opening through which the three loop members extend and four contiguous sides,
   a rectangular or ovular shape comprising three openings each comprising a respective loop member of the three loop members extending therethrough,
   a cylindrical shape comprising three passageways therethrough, the three passageways each comprising a respective loop member of the three loop members extending therethrough, and
   a tapered ring comprising an annular opening through which the three loop members extend and a tapered height extending across a diameter of annular opening.
11. The apparatus of embodiment 1 or embodiment 6, further comprising:
   a plurality of electrodes affixed to the three loop members, each electrode of the plurality of electrodes comprising a surface characterized by roughness parameter *Ra* representing an arithmetical mean deviation of a profile of the surface, where *Ra* measures from about 0.3 micrometers to about 0.4 micrometers.
12. The apparatus of embodiment 6, further comprising:
   at least one pull wire extending through the tubular member and attached to a distal portion of the tubular member so that when the pull wire is retracted proximally, the distal portion and the three loop members are bent at an angle with respect to the longitudinal axis.
13. A method comprising:
   shaping a first loop member, a second loop member, and a third loop member to each form a respective loop;
   coupling a respective pair of ends for each of the first loop member, the second loop member, and the third loop member to a distal portion of an elongated shaft;
   overlapping the first loop member, the second loop member, and the third loop member at a common distal vertex distal to the distal portion of the elongated shaft such that when the first, second, and third loop members are unconstrained, a majority of the first loop member is non-coplanar with a majority of at least one of the second loop member and the third loop member;
   pressing the majority of the first loop member, the majority of the second loop member, and the majority of the third loop member in contact with a planar surface via manipulation of the elongated shaft; and
   contacting the first, second and third loop members with a planar surface to align the majority of the first loop member, the majority of the second loop member, and the majority of the third loop member with the planar surface.
14. The method of embodiment 13, further comprising:
   positioning the first loop member, second loop member, third loop member, and distal portion of the elongated shaft within an intravascular catheter while a proximal portion of the elongated shaft extends proximally from the intravascular catheter;
      moving the first loop member, second loop member, and third loop member out of a distal end of the catheter via manipulation of the proximal portion of the elongated shaft; and
   pressing the majority of the first loop member, the majority of the second loop member, and the majority of the third loop member in contact with the planar surface via manipulation of the proximal portion of the elongated shaft.
15. The method of embodiment 13, further comprising:
   shaping a first support frame to define a first looped path such that the first support frame comprises a cross sectional shape orthogonal to the first looped path that varies along the first looped path;
   positioning the first support frame in the first loop member;
   shaping a second support frame to define a second looped path such that the second support frame comprises a cross sectional shape orthogonal to the second looped path that varies along the second looped path;
   positioning the second support frame in the second loop member;
   shaping a third support frame to define a third looped path such that the third support frame comprises a cross sectional shape orthogonal to the third looped path that varies along the third looped path;
   positioning the third support frame in the third loop member; and
   affixing the first, second, and third support frames to the distal portion of the elongated shaft at each end of the respective pair of ends of the first loop member, the second loop member, and the third loop member.
16. The method of embodiment 15, further comprising:
   at least partially surrounding the first support frame in an inner tubular housing;
   positioning a plurality of electrical conductors adjacent to the first support frame and outside of the inner tubular housing;
   at least partially surrounding the inner tubular housing and the plurality of electrical conductors with an outer tubular housing; and
   bonding the outer tubular housing to the inner tubular housing.
17. The method of embodiment 15, further comprising:
   positioning the first support frame, the second support frame, and the third support frame such that the first looped path defines a first plane intersecting at least one of a second plane defined by the second looped path and a third plane defined by the third looped path;
   shaping a first pair of parallel segments in the first support frame, a second pair of parallel segments in the second support frame, and a third pair of parallel segments in the third support frame; and
   moving a majority of a length of each segment of the first pair of parallel segments, the second pair of parallel segments, and the third pair of parallel segments into alignment parallel to the planar surface via manipulation of the elongated shaft.
18. The method of embodiment 13, further comprising:
   affixing a plurality of electrodes to the first, second, and third loop members;
   abrading at least a portion of a surface of each electrode of the plurality of electrodes; and
   swaging each electrode of the plurality of electrodes.

## Claims

1. An apparatus comprising:
an elongated shaft comprising a proximal portion and a distal portion, the elongated shaft configured to be manipulated at the proximal portion to position the distal portion into a heart of a patient, the elongated shaft defining a longitudinal axis of the apparatus; and
an end effector disposed proximate the distal portion of the elongated shaft, the end effector comprising three loop members overlapping at a common distal vertex along the longitudinal axis, each of the three loop members comprising respective pair of ends affixed to the distal portion of the elongated shaft, the end effector configured to expand to an unconstrained configuration when unconstrained to define three different planes for the respective three loop members, so that when distal end of the elongated shaft is deflected at an angle relative to the longitudinal axis and the loop members are positioned against a planar surface, a majority of a length of each of the three loop members becomes contiguous to the planar surface in a flattened configuration.

2. The apparatus of claim 1,
each of the three loop members respectively comprising a support frame extending through a respective loop member of the three loop members and affixed to the distal portion of the elongated shaft at each end of the respective pair of ends of the respective loop member,
each of the respective support frames defining a respective looped path of the respective loop member when the end effector is in the unconstrained configuration, and
each of the respective support frames comprising a respective cross sectional shape orthogonal to the respective looped path, each of the respective cross sectional shapes varying along the respective looped path.

3. The apparatus of claim 2,
each of the respective support frames comprising:
a respective pair of parallel segments, each segment in the respective pair of parallel segments comprising a respective length,
a majority of the respective length of each segment in each of the respective pairs of parallel segments being approximately coplanar with a majority of the respective length of each of the other segments in each of the respective pairs of parallel segments when the end effector is in the flattened configuration, and
the majority of the respective lengths the segments of at least one of the respective pairs of parallel segments being non-coplanar with the majority of the respective length of at least one segment of the other respective pairs of parallel segments when the end effector is in the unconstrained configuration.

4. The apparatus of claim 1, further comprising:
a mechanical linkage binding the three loop members at the distal vertex, the mechanical linkage comprising at least one of:
a rectangular or ovular shape comprising an opening through which the three loop members extend and a side comprising a seam,
a rectangular or ovular shape comprising an opening through which the three loop members extend and four contiguous sides,
a rectangular or ovular shape comprising three openings each comprising a respective loop member of the three loop members extending therethrough,
a cylindrical shape comprising three passageways therethrough, the three passageways each comprising a respective loop member of the three loop members extending therethrough, and
a tapered ring comprising an annular opening through which the three loop members extend and a tapered height extending across a diameter of annular opening.

5. The apparatus of claim 1, further comprising:
at least one pull wire extending through the elongated shaft and attached to the distal portion of the elongated shaft so that when the pull wire is retracted toward the proximal portion relative to the elongated shaft, the distal portion and the end effector are bent at an angle with respect to the longitudinal axis.

6. An apparatus for a mapping catheter comprising:
a tubular member extending along a longitudinal axis;
a first loop member comprising two spine members extending from the tubular member and connected to an arcuate member, the first loop member arrayed on a first plane;
a second loop member comprising two spine members extending from the tubular member and connected to an arcuate member, the second loop member arrayed on a second plane that intersects with the first plane;
a third loop member comprising two spine members extending from the tubular member and connected to an arcuate member, the third loop member arrayed on a third plane that intersects with the first plane and the second plane so that majority of the length each of the three loop members is non-coplanar with the majority of the length of at least one of the other three loop members in an unconstrained configuration; and
each of the first, second and third loop members including a plurality of electrodes disposed thereon each loop member.

7. The apparatus of claim 6,
each of the three loop members respectively comprising a support frame extending through a respective loop member of the three loop members and affixed to a distal portion of the tubular member,
each of the respective support frames defining a respective looped path of the respective loop member, and
each of the respective support frames comprising a respective cross sectional shape orthogonal to the respective looped path, each of the respective cross sectional shapes varying along the respective looped path.

8. The apparatus of claim 2 or claim 7,
the three loop members each comprising:
an inner tubular housing surrounding at least a portion of the respective support frame;
an outer tubular housing surrounding at least a portion of the inner tubular housing and bonded to the inner tubular housing; and
a plurality of electrical conductors disposed at least partially within the outer tubular housing and outside of the inner tubular housing.

9. The apparatus of claim 7,
the three loop members movable to a flattened configuration when the loop members are positioned against a planar surface, a majority of a length of each of the three loop members being contiguous to the planar surface in the flattened configuration, and
each of the respective support frames comprising:
a respective pair of parallel segments, each segment in the respective pair of parallel segments comprising a respective length,
a majority of the respective length of each segment in each of the respective pairs of parallel segments being approximately coplanar with a majority of the respective length of each of the other segments in each of the respective pairs of parallel segments when the three loop members are in the flattened configuration, and
the majority of the respective lengths the segments of at least one of the respective pairs of parallel segments being non-coplanar with the majority of the respective length of at least one segment of the other respective pairs of parallel segments when the three loop members are in the unconstrained configuration.

10. The apparatus of claim 6, further comprising:
a mechanical linkage binding the arcuate members of the three loop members, the mechanical linkage comprising at least one of:
a rectangular or ovular shape comprising an opening through which the three loop members extend and a side comprising a seam,
a rectangular or ovular shape comprising an opening through which the three loop members extend and four contiguous sides,
a rectangular or ovular shape comprising three openings each comprising a respective loop member of the three loop members extending therethrough,
a cylindrical shape comprising three passageways therethrough, the three passageways each comprising a respective loop member of the three loop members extending therethrough, and
a tapered ring comprising an annular opening through which the three loop members extend and a tapered height extending across a diameter of annular opening.

11. The apparatus of claim 1 or claim 6, further comprising:
a plurality of electrodes affixed to the three loop members, each electrode of the plurality of electrodes comprising a surface **characterized by** roughness parameter *Ra* representing an arithmetical mean deviation of a profile of the surface, where *Ra* measures from about 0.3 micrometers to about 0.4 micrometers.

12. The apparatus of claim 6, further comprising:
at least one pull wire extending through the tubular member and attached to a distal portion of the tubular member so that when the pull wire is retracted proximally, the distal portion and the three loop members are bent at an angle with respect to the longitudinal axis.
